# EUROPEAN PATENT SPECIFICATION

(11) **EP 2 035 581 B1**
(45) Date of publication and mention of the grant of the patent: **26.09.2012**
(21) Application number: 07796305.6
(22) Date of filing: 21.06.2007
(51) Int. Cl.: C12Q 1/68

(54) **DNA COMPOSITION AGAINST TUMOR STROMAL ANTIGEN FAP AND METHODS OF USE THEREOF**
DNA-ZUSAMMENSETZUNG GEGEN TUMOR-STROMA-ANTIGEN FAP UND VERWENDUNGSVERFAHREN
COMPOSITION D'ADN CONTRE L'ANTIGENE STROMAL TUMORAL FAP ET PROCEDEÉS D'UTILISATION DE CELLE-CI

(30) Priority: 21.06.2006 US 815316 P
(43) Date of publication of application: 18.03.2009
(73) Proprietor: THE SCRIPPS RESEARCH INSTITUTE, La Jolla, CA 92037 (US)
(72) Inventor: REISFELD, Ralph, A., La Jolla, California 92037 (US); LOEFFLER, Markus, San Diego, California 92121 (US); KRÜGER, Jörg, A., San Diego, California 92109 (US); NIETHAMMER, Andreas, G., La Jolla, California 92037 (US)
(74) Representative: Schnappauf, Georg
(86) International application number: PCT/US2007/014440
(87) International publication number: WO 2007/149518

(56) References cited:
- WO-A-02/083171
- WO-A-03/064612
- US-A1- 2002 034 789
- US-A1- 2003 157 534
- US-A1- 2006 088 532
- LEE JAEWOO ET AL: "Tumor immunotherapy targeting fibroblast activation protein, a product expressed in tumor-associated fibroblasts." CANCER RESEARCH 1 DEC 2005, vol. 65, no. 23, 1 December 2005 (2005-12-01), pages 11156-11163, XP002549066 ISSN: 0008-5472
- FASSNACHT MARTIN ET AL: "Induction of CD4(+) and CD8(+) T-cell responses to the human stromal antigen, fibroblast activation protein: implication for cancer immunotherapy." CLINICAL CANCER RESEARCH : AN OFFICIAL JOURNAL OF THE AMERICAN ASSOCIATION FOR CANCER RESEARCH 1 AUG 2005, vol. 11, no. 15, 1 August 2005 (2005-08-01), pages 5566-5571, XP002549067 ISSN: 1078-0432
- NIETHAMMER ANDREAS G ET AL: "A DNA vaccine against VEGF receptor 2 prevents effective angiogenesis and inhibits tumor growth." NATURE MEDICINE DEC 2002, vol. 8, no. 12, December 2002 (2002-12), pages 1369-1375, XP002968984 ISSN: 1078-8956
- LOEFFLER MARKUS ET AL: "Targeting tumor-associated fibroblasts improves cancer chemotherapy by increasing intratumoral drug uptake." THE JOURNAL OF CLINICAL INVESTIGATION JUL 2006, vol. 116, no. 7, July 2006 (2006-07), pages 1955-1962, XP002549068 ISSN: 0021-9738
- CHENG ET AL.: 'Promotion of Tumor Growth by Murine Fibroblast Activation Protein, a Serine Protease, in an Animal Model' CANCER RESEARCH vol. 62, August 2002, pages 4767 - 4772, XP008100989
- HATTORI ET AL.: 'Enhanced DNA vaccine potency by mannosylated lipoplex after intraperitoneal administration' THE JOURNAL OF GENE MEDICINE vol. 8, no. 7, April 2006, pages 824 - 834, XP008100993
- XIANG ET AL.: 'A Dual-Function DNA Vaccine Encoding Carcinoembryonic Antigen and CD40 Ligand Trimer Induces T Cell-Mediated Protective Immunity Against Colon Cancer in Carcinoembryonic Antigen-Transgenic Mice' THE JOURNAL OF IMMUNOLOGY vol. 167, August 2001, pages 4560 - 4565, XP002964994
- SHERMAN-BAUST ET AL.: 'Remodeling of the extracellular matrix through overexpression of collagen VI contributes to cisplastin resistance in ovarian cancer cells' CANCER CELL vol. 3, no. 4, April 2003, pages 377 - 386, XP003006271
- PARK ET AL.: 'Fibroblast Activation Protein, a Dual Specificity Serine Protease Expressed in Reactive Human Tumor Stromal Fibroblasts' THE JOURNAL OF BIOLOGICAL CHEMISTRY vol. 274, no. 51, December 1999, pages 36505 - 36512, XP002163938

### SEQUENCE LISTING

<110> REISFELD, Ralph A. LOEFFLER, Markus KRÜGER, Jörg A. NIETHAMMER, Andreas G.
<120> DNA COMPOSITION AGAINST TUMOR STROMAL
   ANTIGEN FAP AND METHODS OF USE THEREOF
<130> TSRI 951.1 PCT
<150> US 60/815,316
   <151> 2006-06-21
<160> 16
<170> FastSEQ for Windows Version 4.0
<210> 1
   <211> 2648
   <212> DNA
   <213> homo sapiens
<400> 1
   ctgaagacag aattagctaa ctttcaaaaa catctggaaa aatgaagact tgggtaaaaa 60
   tcgtatttgg agttgccacc tctgctgtgc ttgccttatt ggtgatgtgc attgtcttac 120
   gcccttcaag agttcataac tctgaagaaa atacaatgag agcactcaca ctgaaggata 180
   ttttaaatgg aacattttct tataaaacat tttttccaaa ctggatttca ggacaagaat 240
   atcttcatca atctgcagat aacaatatag tactttataa tattgaaaca ggacaatcat 300
   ataccatttt gagtaataga accatgaaaa gtgtgaatgc ttcaaattac ggcttatcac 360
   ctgatcggca atttgtatat ctagaaagtg attattcaaa gctttggaga tactcttaca 420
   cagcaacata ttacatctat gaccttagca atggagaatt tgtaagagga aatgagcttc 480
   ctcgtccaat tcagtattta tgctggtcgc ctgttgggag taaattagca tatgtctatc 540
   aaaacaatat ctatttgaaa caaagaccag gagatccacc ttttcaaata acatttaatg 600
   gaagagaaaa taaaatattt aatggaatcc cagactgggt ttatgaagag gaaatgcttg 660
   ctacaaaata tgctctctgg tggtctccta atggaaaatt tttggcatat gcggaattta 720
   atgatacgga tataccagtt attgcctatt cctattatgg cgatgaacaa tatcctagaa 780
   caataaatat tccataccca aaggctggag ctaagaatcc cgttgttcgg atatttatta 840
   tcgataccac ttaccctgcg tatgtaggtc cccaggaagt gcctgttcca gcaatgatag 900
   cctcaagtga ttattatttc agttggctca cgtgggttac tgatgaacga gtatgtttgc 960
   agtggctaaa aagagtccag aatgtttcgg tcctgtctat atgtgacttc agggaagact 1020
   ggcagacatg ggattgtcca aagacccagg agcatataga agaaagcaga actggatggg 1080
   ctggtggatt ctttgtttca acaccagttt tcagctatga tgccatttcg tactacaaaa 1140
   tatttagtga caaggatggc tacaaacata ttcactatat caaagacact gtggaaaatg 1200
   ctattcaaat tacaagtggc aagtgggagg ccataaatat attcagagta acacaggatt 1260
   cactgtttta ttctagcaat gaatttgaag aataccctgg aagaagaaac atctacagaa 1320
   ttagcattgg aagctatcct ccaagcaaga agtgtgttac ttgccatcta aggaaagaaa 1380
   ggtgccaata ttacacagca agtttcagcg actacgccaa gtactatgca cttgtctgct 1440
   acggcccagg catccccatt tccacccttc atgatggacg cactgatcaa gaaattaaaa 1500
   tcctggaaga aaacaaggaa ttggaaaatg ctttgaaaaa tatccagctg cctaaagagg 1560
   aaattaagaa acttgaagta gatgaaatta ctttatggta caagatgatt cttcctcctc 1620
   aatttgacag atcaaagaag tatcccttgc taattcaagt gtatggtggt ccctgcagtc 1680
   agagtgtaag gtctgtattt gctgttaatt ggatatctta tcttgcaagt aaggaaggga 1740
   tggtcattgc cttggtggat ggtcgaggaa cagctttcca aggtgacaaa ctcctctatg 1800
   cagtgtatcg aaagctgggt gtttatgaag ttgaagacca gattacagct gtcagaaaat 1860
   tcatagaaat gggtttcatt gatgaaaaaa gaatagccat atggggctgg tcctatggag 1920
   gatacgtttc atcactggcc cttgcatctg gaactggtct tttcaaatgt ggtatagcag 1980
   tggctccagt ctccagctgg gaatattacg cgtctgtcta cacagagaga ttcatgggtc 2040
   tcccaacaaa ggatgataat cttgagcact ataagaattc aactgtgatg gcaagagcag 2100
   aatatttcag aaatgtagac tatcttctca tccacggaac agcagatgat aatgtgcact 2160
   ttcaaaactc agcacagatt gctaaagctc tggttaatgc acaagtggat ttccaggcaa 2220
   tgtggtactc tgaccagaac cacggcttat ccggcctgtc cacgaaccac ttatacaccc 2280
   acatgaccca cttcctaaag cagtgtttct ctttgtcaga ctaaaaacga tgcagatgca 2340
   agcctgtatc agaatctgaa aaccttatat aaacccctca gacagtttgc ttattttatt 2400
   ttttatgttg taaaatgcta gtataaacaa acaaattaat gttgttctaa aggctgttaa 2460
   aaaaaagatg aggactcaga agttcaagct aaatattgtt tacattttct ggtactctgt 2520
   gaaagaagag aaaagggagt catgcatttt gctttggaca cagtgtttta tcacctgttc 2580
   atttgaagaa aaataataaa gtcagaagtt caagtgcgaa aaaaaaaaaa aaaaaaaaaa 2640
   aaaaaaaa                                                          2648
<210> 2
   <211> 760
   <212> PRT
   <213> homo sapiens
<400> 2
   Met Lys Thr Trp Val Lys Ile Val Phe Gly Val Ala Thr Ser Ala Val
    1               5                  10                  15
   Leu Ala Leu Leu Val Met Cys Ile Val Leu Arg Pro Ser Arg Val His
               20                  25                  30
   Asn Ser Glu Glu Asn Thr Met Arg Ala Leu Thr Leu Lys Asp Ile Leu
           35                  40                  45
   Asn Gly Thr Phe Ser Tyr Lys Thr Phe Phe Pro Asn Trp Ile Ser Gly
       50                  55                  60
   Gln Glu Tyr Leu His Gln Ser Ala Asp Asn Asn Ile Val Leu Tyr Asn
   65                  70                  75                  80
   Ile Glu Thr Gly Gln Ser Tyr Thr Ile Leu Ser Asn Arg Thr Met Lys
                   85                  90                  95
   Ser Val Asn Ala Ser Asn Tyr Gly Leu Ser Pro Asp Arg Gln Phe Val
               100                 105                 110
   Tyr Leu Glu Ser Asp Tyr Ser Lys Leu Trp Arg Tyr Ser Tyr Thr Ala
           115                 120                 125
   Thr Tyr Tyr Ile Tyr Asp Leu Ser Asn Gly Glu Phe Val Arg Gly Asn
       130                 135                 140
   Glu Leu Pro Arg Pro Ile Gln Tyr Leu Cys Trp Ser Pro Val Gly Ser
   145                 150                 155                 160
   Lys Leu Ala Tyr Val Tyr Gln Asn Asn Ile Tyr Leu Lys Gln Arg Pro
                   165                 170                 175
   Gly Asp Pro Pro Phe Gln Ile Thr Phe Asn Gly Arg Glu Asn Lys Ile
               180                 185                 190
   Phe Asn Gly Ile Pro Asp Trp Val Tyr Glu Glu Glu Met Leu Ala Thr
           195                 200                 205
   Lys Tyr Ala Leu Trp Trp Ser Pro Asn Gly Lys Phe Leu Ala Tyr Ala
       210                 215                 220
   Glu Phe Asn Asp Thr Asp Ile Pro Val Ile Ala Tyr Ser Tyr Tyr Gly
   225                 230                 235                 240
   Asp Glu Gln Tyr Pro Arg Thr Ile Asn Ile Pro Tyr Pro Lys Ala Gly
                   245                 250                 255
   Ala Lys Asn Pro Val Val Arg Ile Phe Ile Ile Asp Thr Thr Tyr Pro
               260                 265                 270
   Ala Tyr Val Gly Pro Gln Glu Val Pro Val Pro Ala Met Ile Ala Ser
           275                 280                 285
   Ser Asp Tyr Tyr Phe Ser Trp Leu Thr Trp Val Thr Asp Glu Arg Val
       290                 295                 300
   Cys Leu Gln Trp Leu Lys Arg Val Gln Asn Val Ser Val Leu Ser Ile
   305                 310                 315                 320
   Cys Asp Phe Arg Glu Asp Trp Gln Thr Trp Asp Cys Pro Lys Thr Gln
                           325                             330                             335
   Glu His Ile Glu Glu Ser Arg Thr Gly Trp Ala Gly Gly Phe Phe Val
                   340                             345                             350
   Ser Thr Pro Val Phe Ser Tyr Asp Ala Ile Ser Tyr Tyr Lys Ile Phe
           355                             360                             365
   Ser Asp Lys Asp Gly Tyr Lys His Ile His Tyr Ile Lys Asp Thr Val
       370                             375                             380
   Glu Asn Ala Ile Gln Ile Thr Ser Gly Lys Trp Glu Ala Ile Asn Ile
   385                             390                             395                             400
   Phe Arg Val Thr Gln Asp Ser Leu Phe Tyr Ser Ser Asn Glu Phe Glu
                           405                             410                             415
   Glu Tyr Pro Gly Arg Arg Asn Ile Tyr Arg Ile Ser Ile Gly Ser Tyr
                   420                             425                             430
   Pro Pro Ser Lys Lys Cys Val Thr Cys His Leu Arg Lys Glu Arg Cys
           435                             440                             445
   Gln Tyr Tyr Thr Ala Ser Phe Ser Asp Tyr Ala Lys Tyr Tyr Ala Leu
       450                             455                             460
   Val Cys Tyr Gly Pro Gly Ile Pro Ile Ser Thr Leu His Asp Gly Arg
   465                             470                             475                             480
   Thr Asp Gln Glu Ile Lys Ile Leu Glu Glu Asn Lys Glu Leu Glu Asn
                           485                             490                             495
   Ala Leu Lys Asn Ile Gln Leu Pro Lys Glu Glu Ile Lys Lys Leu Glu
                   500                             505                             510
   Val Asp Glu Ile Thr Leu Trp Tyr Lys Met Ile Leu Pro Pro Gln Phe
           515                             520                             525
   Asp Arg Ser Lys Lys Tyr Pro Leu Leu Ile Gln Val Tyr Gly Gly Pro
       530                             535                             540
   Cys Ser Gln Ser Val Arg Ser Val Phe Ala Val Asn Trp Ile Ser Tyr
   545                             550                             555                             560
   Leu Ala Ser Lys Glu Gly Met Val Ile Ala Leu Val Asp Gly Arg Gly
                           565                             570                             575
   Thr Ala Phe Gln Gly Asp Lys Leu Leu Tyr Ala Val Tyr Arg Lys Leu
                   580                             585                             590
   Gly Val Tyr Glu Val Glu Asp Gln Ile Thr Ala Val Arg Lys Phe Ile
           595                             600                             605
   Glu Met Gly Phe Ile Asp Glu Lys Arg Ile Ala Ile Trp Gly Trp Ser
       610                             615                             620
   Tyr Gly Gly Tyr Val Ser Ser Leu Ala Leu Ala Ser Gly Thr Gly Leu
   625                             630                             635                             640
   Phe Lys Cys Gly Ile Ala Val Ala Pro Val Ser Ser Trp Glu Tyr Tyr
                           645                             650                             655
   Ala Ser Val Tyr Thr Glu Arg Phe Met Gly Leu Pro Thr Lys Asp Asp
                   660                             665                             670
   Asn Leu Glu His Tyr Lys Asn Ser Thr Val Met Ala Arg Ala Glu Tyr
           675                             680                             685
   Phe Arg Asn Val Asp Tyr Leu Leu Ile His Gly Thr Ala Asp Asp Asn
       690                             695                             700
   Val His Phe Gln Asn Ser Ala Gln Ile Ala Lys Ala Leu Val Asn Ala
   705                             710                             715                             720
   Gln Val Asp Phe Gln Ala Met Trp Tyr Ser Asp Gln Asn His Gly Leu
                           725                             730                             735
   Ser Gly Leu Ser Thr Asn His Leu Tyr Thr His Met Thr His Phe Leu
                   740                             745                             750
   Lys Gln Cys Phe Ser Leu Ser Asp
           755                             760
<210> 3
   <211> 2615
   <212> DNA
   <213> mus musculus
<400> 3
   ccacgcgtcc gcacagatgc ggtgacccac gctgtgcagt gagaatcagc taacttgcaa 60
   aaacatctgg aaaaatgaag acatggctga aaactgtctt tggagttacc accctggctg 120
   cgcttgcttt agtggtgata tgcattgtct tacgtccctc aagagtttac aaacctgaag 180
   gaaacacaaa gagagctctt accttgaagg atattttaaa tggaacattc tcatataaaa 240
   catattttcc caactggatt tcagaacaag aatatcttca tcaatctgag gatgataaca 300
   tagtatttta taatattgaa acaagagaat catatatcat tttgagtaat agcaccatga 360
   aaagtgtgaa tgctacagat tatggtttgt cacctgatcg gcaatttgtg tatctagaaa 420
   gtgattattc aaagctctgg cgatattcat acacagcgac atactacatc tacgaccttc 480
   agaatgggga atttgtaaga ggatacgagc tccctcgtcc aattcagtat ctatgctggt 540
   cgcctgttgg gagtaaatta gcatatgtat atcaaaacaa tatttatttg aaacaaagac 600
   caggagatcc accttttcaa ataacttata ctggaagaga aaatagaata tttaatggaa 660
   taccagactg ggtttatgaa gaggaaatgc ttgccacaaa atatgctctt tggtggtctc 720
   cagatggaaa atttttggca tatgtagaat ttaatgattc agatatacca attattgcct 780
   attcttatta tggtgatgga cagtatccta gaactataaa tattccatat ccaaaggctg 840
   gggctaagaa tccggttgtt cgtgttttta ttgttgacac cacctaccct caccacgtgg 900
   gcccaatgga agtgccagtt ccagaaatga tagcctcaag tgactattat ttcagctggc 960
   tcacatgggt gtccagtgaa cgagtatgct tgcagtggct aaaaagagtg cagaatgtct 1020
   cagtcctgtc tatatgtgat ttcagggaag actggcatgc atgggaatgt ccaaagaacc 1080
   aggagcatgt agaagaaagc agaacaggat gggctggtgg attctttgtt tcgacaccag 1140
   cttttagcca ggatgccact tcttactaca aaatatttag cgacaaggat ggttacaaac 1200
   atattcacta catcaaagac actgtggaaa atgctattca aattacaagt ggcaagtggg 1260
   aggccatata tatattccgc gtaacacagg attcactgtt ttattctagc aatgaatttg 1320
   aaggttaccc tggaagaaga aacatctaca gaattagcat tggaaactct cctccgagca 1380
   agaagtgtgt tacttgccat ctaaggaaag aaaggtgcca atattacaca gcaagtttca 1440
   gctacaaagc caagtactat gcactcgtct gctatggccc tggcctcccc atttccaccc 1500
   tccatgatgg ccgcacagac caagaaatac aagtattaga agaaaacaaa gaactggaaa 1560
   attctctgag aaatatccag ctgcctaaag tggagattaa gaagctcaaa gacgggggac 1620
   tgactttctg gtacaagatg attctgcctc ctcagtttga cagatcaaag aagtacccct 1680
   tgctaattca agtgtatggt ggtccttgta gccagagtgt taagtctgtg tttgctgtta 1740
   attggataac ttatctcgca agtaaggagg ggatagtcat tgccctggta gatggtcggg 1800
   gcactgcttt ccaaggtgac aaattcctgc atgccgtgta tcgaaaactg ggtgtatatg 1860
   aagttgagga ccagctcaca gctgtcagaa aattcataga aatgggtttc attgatgaag 1920
   aaagaatagc catatggggc tggtcctacg gaggttatgt ttcatccctg gcccttgcat 1980
   ctggaactgg tcttttcaaa tgtggcatag cagtggctcc agtctccagc tgggaatatt 2040
   acgcatctat ctactcagag agattcatgg gcctcccaac aaaggacgac aatctcgaac 2100
   actataaaaa ttcaactgtg atggcaagag cagaatattt cagaaatgta gactatcttc 2160
   tcatccacgg aacagcagat gataatgtgc actttcagaa ctcagcacag attgctaaag 2220
   ctttggttaa tgcacaagtg gatttccagg cgatgtggta ctctgaccag aaccatggta 2280
   tattatctgg gcgctcccag aatcatttat atacccacat gacgcacttc ctcaagcaat 2340
   gcttttcttt atcagactga accaatgcaa gtactagcgt gtaggacagt ctgaaggctt 2400
   cacatggacg acttggacca tgaatattgt aaaagccgct aaaaatttag gagggctcag 2460
   gaggttaggc tcggtattgt ttacatttcc ctatgctctg tgaaagaaga gaaaagggga 2520
   gccacacatc ttgctttgga cacaatgttt tatcacctgt tcattgccgg gtagcggaaa 2580
   taaagtcaga tgctcaggtt aaaaaaaaaa aaaaa                            2615
<210> 4
   <211> 761
   <212> PRT
   <213> mus musculus
<400> 4
   Met Lys Thr Trp Leu Lys Thr Val Phe Gly Val Thr Thr Leu Ala Ala
   1               5                   10                  15
   Leu Ala Leu Val Val Ile Cys Ile Val Leu Arg Pro Ser Arg Val Tyr
               20                  25                  30
   Lys Pro Glu Gly Asn Thr Lys Arg Ala Leu Thr Leu Lys Asp Ile Leu
           35                  40                  45
   Asn Gly Thr Phe Ser Tyr Lys Thr Tyr Phe Pro Asn Trp Ile Ser Glu
       50                  55                  60
   Gln Glu Tyr Leu His Gln Ser Glu Asp Asp Asn Ile Val Phe Tyr Asn
   65                  70                  75                  80
   Ile Glu Thr Arg Glu Ser Tyr Ile Ile Leu Ser Asn Ser Thr Met Lys
                   85                  90                  95
   Ser Val Asn Ala Thr Asp Tyr Gly Leu Ser Pro Asp Arg Gln Phe Val
               100                 105                 110
   Tyr Leu Glu Ser Asp Tyr Ser Lys Leu Trp Arg Tyr Ser Tyr Thr Ala
           115                 120                 125
   Thr Tyr Tyr Ile Tyr Asp Leu Gln Asn Gly Glu Phe Val Arg Gly Tyr
       130                 135                 140
   Glu Leu Pro Arg Pro Ile Gln Tyr Leu Cys Trp Ser Pro Val Gly Ser
   145                 150                 155                 160
   Lys Leu Ala Tyr Val Tyr Gln Asn Asn Ile Tyr Leu Lys Gln Arg Pro
                   165                 170                 175
   Gly Asp Pro Pro Phe Gln Ile Thr Tyr Thr Gly Arg Glu Asn Arg Ile
               180                 185                 190
   Phe Asn Gly Ile Pro Asp Trp Val Tyr Glu Glu Glu Met Leu Ala Thr
           195                 200                 205
   Lys Tyr Ala Leu Trp Trp Ser Pro Asp Gly Lys Phe Leu Ala Tyr Val
       210                 215                 220
   Glu Phe Asn Asp Ser Asp Ile Pro Ile Ile Ala Tyr Ser Tyr Tyr Gly
   225                 230                 235                 240
   Asp Gly Gln Tyr Pro Arg Thr Ile Asn Ile Pro Tyr Pro Lys Ala Gly
                   245                 250                 255
   Ala Lys Asn Pro Val Val Arg Val Phe Ile Val Asp Thr Thr Tyr Pro
               260                 265                 270
   His His Val Gly Pro Met Glu Val Pro Val Pro Glu Met Ile Ala Ser
           275                 280                 285
   Ser Asp Tyr Tyr Phe Ser Trp Leu Thr Trp Val Ser Ser Glu Arg Val
       290                 295                 300
   Cys Leu Gln Trp Leu Lys Arg Val Gln Asn Val Ser Val Leu Ser Ile
   305                 310                 315                 320
   Cys Asp Phe Arg Glu Asp Trp His Ala Trp Glu Cys Pro Lys Asn Gln
                   325                 330                 335
   Glu His Val Glu Glu Ser Arg Thr Gly Trp Ala Gly Gly Phe Phe Val
               340                 345                 350
   Ser Thr Pro Ala Phe Ser Gln Asp Ala Thr Ser Tyr Tyr Lys Ile Phe
           355                 360                 365
   Ser Asp Lys Asp Gly Tyr Lys His Ile His Tyr Ile Lys Asp Thr Val
       370                 375                 380
   Glu Asn Ala Ile Gln Ile Thr Ser Gly Lys Trp Glu Ala Ile Tyr Ile
   385                 390                 395                 400
   Phe Arg Val Thr Gln Asp Ser Leu Phe Tyr Ser Ser Asn Glu Phe Glu
                   405                 410                 415
   Gly Tyr Pro Gly Arg Arg Asn Ile Tyr Arg Ile Ser Ile Gly Asn Ser
               420                 425                 430
   Pro Pro Ser Lys Lys Cys Val Thr Cys His Leu Arg Lys Glu Arg Cys
           435                 440                 445
   Gln Tyr Tyr Thr Ala Ser Phe Ser Tyr Lys Ala Lys Tyr Tyr Ala Leu
       450                 455                 460
   Val Cys Tyr Gly Pro Gly Leu Pro Ile Ser Thr Leu His Asp Gly Arg
   465                 470                 475                 480
   Thr Asp Gln Glu Ile Gln Val Leu Glu Glu Asn Lys Glu Leu Glu Asn
                   485                 490                 495
   Ser Leu Arg Asn Ile Gln Leu Pro Lys Val Glu Ile Lys Lys Leu Lys
               500                 505                 510
   Asp Gly Gly Leu Thr Phe Trp Tyr Lys Met Ile Leu Pro Pro Gln Phe
           515                 520                 525
   Asp Arg Ser Lys Lys Tyr Pro Leu Leu Ile Gln Val Tyr Gly Gly Pro
       530                 535                 540
   Cys Ser Gln Ser Val Lys Ser Val Phe Ala Val Asn Trp Ile Thr Tyr
   545                 550                 555                 560
   Leu Ala Ser Lys Glu Gly Ile Val Ile Ala Leu Val Asp Gly Arg Gly
                   565                 570                 575
   Thr Ala Phe Gln Gly Asp Lys Phe Leu His Ala Val Tyr Arg Lys Leu
               580                 585                 590
   Gly Val Tyr Glu Val Glu Asp Gln Leu Thr Ala Val Arg Lys Phe Ile
           595                 600                 605
   Glu Met Gly Phe Ile Asp Glu Glu Arg Ile Ala Ile Trp Gly Trp Ser
       610                 615                 620
   Tyr Gly Gly Tyr Val Ser Ser Leu Ala Leu Ala Ser Gly Thr Gly Leu
   625                 630                 635                 640
   Phe Lys Cys Gly Ile Ala Val Ala Pro Val Ser Ser Trp Glu Tyr Tyr
                   645                 650                 655
   Ala Ser Ile Tyr Ser Glu Arg Phe Met Gly Leu Pro Thr Lys Asp Asp
               660                 665                 670
   Asn Leu Glu His Tyr Lys Asn Ser Thr Val Met Ala Arg Ala Glu Tyr
           675                 680                 685
   Phe Arg Asn Val Asp Tyr Leu Leu Ile His Gly Thr Ala Asp Asp Asn
       690                 695                 700
   Val His Phe Gln Asn Ser Ala Gln Ile Ala Lys Ala Leu Val Asn Ala
   705                 710                 715                 720
   Gln Val Asp Phe Gln Ala Met Trp Tyr Ser Asp Gln Asn His Gly Ile
                   725                 730                 735
   Leu Ser Gly Arg Ser Gln Asn His Leu Tyr Thr His Met Thr His Phe
               740                 745                 750
   Leu Lys Gln Cys Phe Ser Leu Ser Asp
           755                 760
<210> 5
   <211> 814
   <212> DNA
   <213> homo sapiens
<400> 5
   atcactctct ttaatcacta ctcacagtaa cctcaactcc tgccacaatg tacaggatgc 60
   aactcctgtc ttgcattgca ctaagtcttg cacttgtcac aaacagtgca cctacttcaa 120
   gttctacaaa gaaaacacag ctacaactgg agcatttact gctggattta cagatgattt 180
   tgaatggaat taataattac aagaatccca aactcaccag gatgctcaca tttaagtttt 240
   acatgcccaa gaaggccaca gaactgaaac atcttcagtg tctagaagaa gaactcaaac 300
   ctctggagga agtgctaaat ttagctcaaa gcaaaaactt tcacttaaga cccagggact 360
   taatcagcaa tatcaacgta atagttctgg aactaaaggg atctgaaaca acattcatgt 420
   gtgaatatgc tgatgagaca gcaaccattg tagaatttct gaacagatgg attacctttt 480
   gtcaaagcat catctcaaca ctgacttgat aattaagtgc ttcccactta aaacgtatca 540
   ggccttctat ttatttaaat atttaaattt tatatttatt gttgaatgta tggtttgcta 600
   cctattgtaa ctattattct taatcttaaa actataaata tggatctttt atgattcttt 660
   ttgtaagccc taggggctct aaaatggttt cacttattta tcccaaaata tttattatta 720
   tgttgaatgt taaatatagt atctatgtag attggttagt aaaactattt aataaatttg 780
   ataaatataa aaaaaaaaaa aaaaaaaaaa aaaa                             814
<210> 6
   <211> 153
   <212> PRT
   <213> homo sapiens
<400> 6
   Met Tyr Arg Met Gln Leu Leu Ser Cys Ile Ala Leu Ser Leu Ala Leu
    1               5                   10                  15
   Val Thr Asn Ser Ala Pro Thr Ser Ser Ser Thr Lys Lys Thr Gln Leu
               20                  25                  30
   Gln Leu Glu His Leu Leu Leu Asp Leu Gln Met Ile Leu Asn Gly Ile
           35                  40                  45
   Asn Asn Tyr Lys Asn Pro Lys Leu Thr Arg Met Leu Thr Phe Lys Phe
       50                  55                  60
   Tyr Met Pro Lys Lys Ala Thr Glu Leu Lys His Leu Gln Cys Leu Glu
   65                  70                  75                  80
   Glu Glu Leu Lys Pro Leu Glu Glu Val Leu Asn Leu Ala Gln Ser Lys
                   85                  90                  95
   Asn Phe His Leu Arg Pro Arg Asp Leu Ile Ser Asn Ile Asn Val Ile
               100                 105                 110
   Val Leu Glu Leu Lys Gly Ser Glu Thr Thr Phe Met Cys Glu Tyr Ala
           115                 120                 125
   Asp Glu Thr Ala Thr Ile Val Glu Phe Leu Asn Arg Trp Ile Thr Phe
       130                 135                 140
   Cys Gln Ser Ile Ile Ser Thr Leu Thr
   145                 150
<210> 7
   <211> 939
   <212> DNA
   <213> mus musculus
<400> 7
   atcacccttg ctaatcactc ctcacagtga cctcaagtcc tgcaggcatg tacagcatgc 60
   agctcgcatc ctgtgtcaca ttgacacttg tgctccttgt caacagcgca cccacttcaa 120
   gctccacttc aagctctaca gcggaagcac agcagcagca gcagcagcag cagcagcagc 180
   agcagcacct ggagcagctg ttgatggacc tacaggagct cctgagcagg atggagaatt 240
   acaggaacct gaaactcccc aggatgctca ccttcaaatt ttacttgccc aagcaggcca 300
   cagaattgaa agatcttcag tgcctagaag atgaacttgg acctctgcgg catgttctgg 360
   atttgactca aagcaaaagc tttcaattgg aagatgctga gaatttcatc agcaatatca 420
   gagtaactgt tgtaaaacta aagggctctg acaacacatt tgagtgccaa ttcgatgatg 480
   agtcagcaac tgtggtggac tttctgagga gatggatagc cttctgtcaa agcatcatct 540
   caacaagccc tcaataacta tgtacctcct gcttacaaca cataaggctc tctatttatt 600
   taaatattta actttaattt atttttggat gtattgttta ctatcttttg taactactag 660
   tcttcagatg ataaatatgg atctttaaag attctttttg taagccccaa gggctcaaaa 720
   atgttttaaa ctatttatct gaaattattt attatattga attgttaaat atcatgtgta 780
   ggtagactca ttaataaaag tatttagatg attcaaatat aaataagctc agatgtctgt 840
   catttttagg acagcacaaa gtaagcgcta aaataacttc tcagttattc ctgtgaactc 900
   tatgttaatc agtgttttca agaaataaag ctctcctct                        939
<210> 8
   <211> 160
   <212> PRT
   <213> mus musculus
<400> 8
   Met Tyr Ser Met Gln Leu Ala Ser Cys Val Thr Leu Thr Leu Val Leu
    1               5                   10                  15
   Leu Val Asn Ser Ala Pro Thr Ser Ser Ser Thr Ser Ser Ser Thr Ala
               20                  25                  30
   Glu Ala Gln Gln Gln Gln Gln Gln Gln Gln Gln Gln Gln Gln His Leu
           35                  40                  45
   Glu Gln Leu Leu Met Asp Leu Gln Glu Leu Leu Ser Arg Met Glu Asn
       50                  55                  60
   Tyr Arg Asn Leu Lys Leu Pro Arg Met Leu Thr Phe Lys Phe Tyr Leu
   65                  70                  75                  80
   Pro Lys Gln Ala Thr Glu Leu Lys Asp Leu Gln Cys Leu Glu Asp Glu
                   85                  90                  95
   Leu Gly Pro Leu Arg His Val Leu Asp Leu Thr Gln Ser Lys Ser Phe
               100                 105                 110
   Gln Leu Glu Asp Ala Glu Asn Phe Ile Ser Asn Ile Arg Val Thr Val
           115                 120                 125
   Val Lys Leu Lys Gly Ser Asp Asn Thr Phe Glu Cys Gln Phe Asp Asp
       130                 135                 140
   Glu Ser Ala Thr Val Val Asp Phe Leu Arg Arg Trp Ile Ala Phe Cys
   145                 150                 155                 160
<210> 9
   <211> 852
   <212> DNA
   <213> homo sapiens
<400> 9
   cttgcagctg cccacctcac cctcagctct ggcctcttac tcaccctcta ccacagacat 60
   ggctcagtca ctggctctga gcctccttat cctggttctg gcctttggca tccccaggac 120
   ccaaggcagt gatggagggg ctcaggactg ttgcctcaag tacagccaaa ggaagattcc 180
   cgccaaggtt gtccgcagct accggaagca ggaaccaagc ttaggctgct ccatcccagc 240
   tatcctgttc ttgccccgca agcgctctca ggcagagcta tgtgcagacc caaaggagct 300
   ctgggtgcag cagctgatgc agcatctgga caagacacca tccccacaga aaccagccca 360
   gggctgcagg aaggacaggg gggcctccaa gactggcaag aaaggaaagg gctccaaagg 420
   ctgcaagagg actgagcggt cacagacccc taaagggcca tagcccagtg agcagcctgg 480
   agccctggag accccaccag cctcaccaac gcttgaagcc tgaacccaag atgcaagaag 540
   gaggctatgc tcaggggccc tggagcagcc accccatgct ggccttgcca cactctttct 600
   cctgctttaa ccaccccatc tgcattccca gctctaccct gcatggctga gctgcccaca 660
   gcaggccagg tccagagaga ccgaggaggg agagtctccc agggagcatg agaggaggca 720
   gcaggactgt ccccttgaag gagaatcatc aggaccctgg acctgatacg gctccccagt 780
   acaccccacc tcttccttgt aaatatgatt tatacctaac tgaataaaaa gctgttctgt 840
   cttcccaccc gc                                                     852
<210> 10
   <211> 134
   <212> PRT
   <213> homo sapiens
<400> 10
   Met Ala Gln Ser Leu Ala Leu Ser Leu Leu Ile Leu Val Leu Ala Phe
    1              5                   10                  15
   Gly Ile Pro Arg Thr Gln Gly Ser Asp Gly Gly Ala Gln Asp Cys Cys
               20                  25                  30
   Leu Lys Tyr Ser Gln Arg Lys Ile Pro Ala Lys Val Val Arg Ser Tyr
           35                  40                  45
   Arg Lys Gln Glu Pro Ser Leu Gly Cys Ser Ile Pro Ala Ile Leu Phe
       50                  55                  60
   Leu Pro Arg Lys Arg Ser Gln Ala Glu Leu Cys Ala Asp Pro Lys Glu
   65                  70                  75                  80
   Leu Trp Val Gln Gln Leu Met Gln His Leu Asp Lys Thr Pro Ser Pro
                   85                  90                  95
   Gln Lys Pro Ala Gln Gly Cys Arg Lys Asp Arg Gly Ala Ser Lys Thr
               100                 105                 110
   Gly Lys Lys Gly Lys Gly Ser Lys Gly Cys Lys Arg Thr Glu Arg Ser
           115                 120                 125
   Gln Thr Pro Lys Gly Pro
       130
<210> 11
   <211> 615
   <212> DNA
   <213> mus musculus
<400> 11
   gaattcggcc aaagaggcct acggccaaag agggctaaac ttgcggctgt ccatctcacc 60
   tacagctctg gtctcatcct caactcaacc acaatcatgg ctcagatgat gactctgagc 120
   ctccttagcc tggtcctggc tctctgcatc ccctggaccc aaggcagtga tggagggggt 180
   caggactgct gccttaagta cagccagaag aaaattccct acagtattgt ccgaggctat 240
   aggaagcaag aaccaagttt aggctgtccc atcccggcaa tcctgttctc accccggaag 300
   cactctaagc ctgagctatg tgcaaaccct gaggaaggct gggtgcagaa cctgatgcgc 360
   cgcctggacc agcctccagc cccagggaaa caaagccccg gctgcaggaa gaaccgggga 420
   acctctaagt ctggaaagaa aggaaagggc tccaagggct gcaagagaac tgaacagaca 480
   cagccctcaa gaggatagcc cagtagcccg cctggagccc aggagatccc ccacgaactt 540
   caagctgggt ggttcacggt ccaactcaca ggcaaagagg gagctagaaa acagactcag 600
   gagccgctag tcgag                                                  615
<210> 12
   <211> 133
   <212> PRT
   <213> mus musculus
<400> 12
   Met Ala Gln Met Met Thr Leu Ser Leu Leu Ser Leu Val Leu Ala Leu
   1               5                   10                  15
   Cys Ile Pro Trp Thr Gln Gly Ser Asp Gly Gly Gly Gln Asp Cys Cys
               20                  25                  30
   Leu Lys Tyr Ser Gln Lys Lys Ile Pro Tyr Ser Ile Val Arg Gly Tyr
           35                  40                  45
   Arg Lys Gln Glu Pro Ser Leu Gly Cys Pro Ile Pro Ala Ile Leu Phe
       50                  55                  60
   Ser Pro Arg Lys His Ser Lys Pro Glu Leu Cys Ala Asn Pro Glu Glu
   65                  70                  75                  80
   Gly Trp Val Gln Asn Leu Met Arg Arg Leu Asp Gln Pro Pro Ala Pro
                   85                  90                  95
   Gly Lys Gln Ser Pro Gly Cys Arg Lys Asn Arg Gly Thr Ser Lys Ser
               100                 105                 110
   Gly Lys Lys Gly Lys Gly Ser Lys Gly Cys Lys Arg Thr Glu Gln Thr
           115                 120                 125
   Gln Pro Ser Arg Gly
       130
<210> 13
   <211> 878
   <212> DNA
   <213> mus musculus
<400> 13
   atcccagccc acgcacagac ccccaacttg cagctgccca cctcaccctc agctctggcc 60
   tcttactcac cctctaccac agacatggct cagtcactgg ctctgagcct ccttatcctg 120
   gttctggcct ttggcatccc caggacccaa ggcagtgatg gaggggctca ggactgttgc 180
   ctcaagtaca gccaaaggaa gattcccgcc aaggttgtcc gcagctaccg gaagcaggaa 240
   ccaagcttag gctgctccat cccagctatc ctgttcttgc cccgcaagcg ctctcaggca 300
   gagctatgtg cagacccaaa ggagctctgg gtgcagcagc tgatgcagca tctggacaag 360
   acaccatccc cacagaaacc agcccagggc tgcaggaagg acaggggggc ctccaagact 420
   ggcaagaaag gaaagggctc caaaggctgc aagaggactg agcggtcaca gacccctaaa 480
   gggccatagc ccagtgagca gcctggagcc ctggagaccc caccagcctc accagcgctt 540
   gaagcctgaa cccaagatgc aagaaggagg ctatgctcag gggccctgga gcagccaccc 600
   catgctggcc ttgccacact ctttctcctg ctttaaccac cccatctgca ttcccagctc 660
   taccctgcat ggctgagctg cccacagcag gccaggtcca gagagaccga ggagggagag 720
   tctcccaggg agcatgagag gaggcagcag gactgtcccc ttgaaggaga atcatcagga 780
   ccctggacct gatacggctc cccagtacac cccacctctt ccttgtaaat atgatttata 840
   cctaactgaa taaaaagctg ttctgtcttc ccacccaa                         878
<210> 14
   <211> 134
   <212> PRT
   <213> mus musculus
<400> 14
   Met Ala Gln Ser Leu Ala Leu Ser Leu Leu Ile Leu Val Leu Ala Phe
   1               5                   10                  15
   Gly Ile Pro Arg Thr Gln Gly Ser Asp Gly Gly Ala Gln Asp Cys Cys
               20                  25                  30
   Leu Lys Tyr Ser Gln Arg Lys Ile Pro Ala Lys Val Val Arg Ser Tyr
           35                  40                  45
   Arg Lys Gln Glu Pro Ser Leu Gly Cys Ser Ile Pro Ala Ile Leu Phe
       50                  55                  60
   Leu Pro Arg Lys Arg Ser Gln Ala Glu Leu Cys Ala Asp Pro Lys Glu
   65                  70                  75                  80
   Leu Trp Val Gln Gln Leu Met Gln His Leu Asp Lys Thr Pro Ser Pro
                   85                  90                  95
   Gln Lys Pro Ala Gln Gly Cys Arg Lys Asp Arg Gly Ala Ser Lys Thr
               100                 105                 110
   Gly Lys Lys Gly Lys Gly Ser Lys Gly Cys Lys Arg Thr Glu Arg Ser
           115                 120                 125
   Gln Thr Pro Lys Gly Pro
       130
<210> 15
   <211> 1816
   <212> DNA
   <213> homo sapiens
<400> 15
   cttctctgcc agaagatacc atttcaactt taacacagca tgatcgaaac atacaaccaa 60
   acttctcccc gatctgcggc cactggactg cccatcagca tgaaaatttt tatgtattta 120
   cttactgttt ttcttatcac ccagatgatt gggtcagcac tttttgctgt gtatcttcat 180
   agaaggttgg acaagataga agatgaaagg aatcttcatg aagattttgt attcatgaaa 240
   acgatacaga gatgcaacac aggagaaaga tccttatcct tactgaactg tgaggagatt 300
   aaaagccagt ttgaaggctt tgtgaaggat ataatgttaa acaaagagga gacgaagaaa 360
   gaaaacagct ttgaaatgca aaaaggtgat cagaatcctc aaattgcggc acatgtcata 420
   agtgaggcca gcagtaaaac aacatctgtg ttacagtggg ctgaaaaagg atactacacc 480
   atgagcaaca acttggtaac cctggaaaat gggaaacagc tgaccgttaa aagacaagga 540
   ctctattata tctatgccca agtcaccttc tgttccaatc gggaagcttc gagtcaagct 600
   ccatttatag ccagcctctg cctaaagtcc cccggtagat tcgagagaat cttactcaga 660
   gctgcaaata cccacagttc cgccaaacct tgcgggcaac aatccattca cttgggagga 720
   gtatttgaat tgcaaccagg tgcttcggtg tttgtcaatg tgactgatcc aagccaagtg 780
   agccatggca ctggcttcac gtcctttggc ttactcaaac tctgaacagt gtcaccttgc 840
   aggctgtggt ggagctgacg ctgggagtct tcataataca gcacagcggt taagcccacc 900
   ccctgttaac tgcctattta taaccctagg atcctcctta tggagaacta tttattatac 960
   actccaaggc atgtagaact gtaataagtg aattacaggt cacatgaaac caaaacgggc 1020
   cctgctccat aagagcttat atatctgaag cagcaacccc actgatgcag acatccagag 1080
   agtcctatga aaagacaagg ccattatgca caggttgaat tctgagtaaa cagcagataa 1140
   cttgccaagt tcagttttgt ttctttgcgt gcagtgtctt tccatggata atgcatttga 1200
   tttatcagtg aagatgcaga agggaaatgg ggagcctcag ctcacattca gttatggttg 1260
   actctgggtt cctatggcct tgttggaggg ggccaggctc tagaacgtct aacacagtgg 1320
   agaaccgaaa cccccccccc cccccccgcc accctctcgg acagttattc attctctttc 1380
   aatctctctc tctccatctc tctctttcag tctctctctc tcaacctctt tcttccaatc 1440
   tctctttctc aatctctctg tttccctttg tcagtctctt ccctccccca gtctctcttc 1500
   tcaatccccc tttctaacac acacacacac acacacacac acacacacac acacacacac 1560
   acacacacac acacacacac agagtcaggc cgttgctagt cagttctctt ctttccaccc 1620
   tgtccctatc tctaccacta tagatgaggg tgaggagtag ggagtgcagc cctgagcctg 1680
   cccactcctc attacgaaat gactgtattt aaaggaaatc tattgtatct acctgcagtc 1740
   tccattgttt ccagagtgaa cttgtaatta tcttgttatt tattttttga ataataaaga 1800
   cctcttaaca ttaaaa                                                 1816
<210> 16
   <211> 261
   <212> PRT
   <213> homo sapiens
<400> 16

## Description

### FIELD OF THE INVENTION

This invention relates to deoxyribonucleic acid (DNA) compositions encoding suitable molecules effective for eliciting an immune response against stromal fibroblast cells in tumors. More particularly this invention relates to DNA compositions encoding for the stromal antigen known as fibroblast activation protein (FAP). This invention also relates to methods of using the DNA composition to inhibit tumor growth and tumor metastases and to enhance cellular uptake of chemotherapeutic agents.

### BACKGROUND OF THE INVENTION

It has become increasingly clear that tumor antigens are elusive targets for cancer therapy. This can be explained by a variety of characteristics unique to tumor cells, including mutations of their antigens and inadequate antigen presentation due to down-regulation of MHC-antigen expression. Moreover, defects in apoptosis signaling pathways, as well as up-regulation of apoptosis-inhibitors such as survivin, XIAP or anti-apoptotic members of the bcl-2 family not only confer resistance to T cell mediated killing, but also to the apoptosis-inducing effect of chemotherapeutic agents.

There is a growing recognition that the stromal compartment plays a crucial role in tumorigenesis and tumor invasion. Stromal cells have been shown to stimulate the transformation of normal epithelial cells, and to interchange growth factors, cytokines and chemoattractants to activate the adjacent extracellular matrix and to induce the selection and expansion of neoplastic cells. In one study, tumor cells injected in mice as a suspension reportedly were not tumorigenic, whereas injection of fragments of solid tumors containing their stroma reportedly led to tumor growth (see Singh et al. J. Exp. Med. 1992; 175:139-146). The activated extracellular matrix apparently confers chemoresistance mediated by β1 integrins, which adhere to fibronectin, leading to the activation of β1 integrin-stimulated tyrosine kinase, which in turn, suppresses chemotherapy-induced apoptosis. This phenomenon has been reported in doxorubicin-sensitive myeloma cells, which developed resistance after adherence to fibronectin. Recent reports indicate that tumor rejection can be achieved by modulating tumor stromal fibroblasts or by distributing the tumor stromal network. In addition, tumor-associated macrophages and fibroblasts reportedly contribute to the local immuno-suppressive environment due to their ability to synthesize proteins, such as VEGF, TGF-β1 and IL-10. VEGF functions as an inhibitor of dendritic cell maturation and thus leads to tolerant T cells. VEGF is also a major factor involved in tumor-angiogenesis. Activation of VEGF leads to upregulation of survivin and many other apoptosis-inhibiting proteins in tumor-endothelial cells, shielding them from the apoptotic effects of chemotherapy. In contrast, inhibition of TGF-β1 can result in tumor eradication and protection against metastasis. IL-10 inhibits the maturation of dendritic cells, and thereby suppresses the cell-mediated antitumor responses mediated by Th1 cells. This effect shifts the dendritic cells towards largely ineffective humoral immune responses.

Fibroblasts are connective tissue cells which secrete an extracellular matrix rich in collagen and other macromolecules. Fibroblasts in the tumor-stroma (i.e., tumor supporting tissue) synthesize fibroblast activation protein (FAP), a type II transmembrane protein that functions as a serine protease. FAP is selectively overexpressed in over 90% of stromal fibroblasts associated with colon, breast and lung carcinomas. Transient overexpression of FAP can also be observed during wound healing and in some fetal mesenchymal tissues. Additionally, this enzyme reportedly cleaves gelatin and type I collagen, and has been implicated in extracellular matrix remodeling. Overexpression of FAP reportedly leads to promotion of tumor growth and increases in metastatic potential, whereas treatment with anti-FAP antibodies inhibits tumor growth. Furthermore, tumor-stromal expression of collagen type I, which is mainly produced by fibroblasts, inversely correlates with intratumor uptake of various compounds, including chemotherapeutic agents, thereby sustaining the involvement of the stromal compartment in chemotherapy resistance.

Vaccines have been utilized to provide a long term protection against a number of disease conditions by very limited administration of a prophylactic agent that stimulates an organism's immune system to destroy disease pathogens before they can proliferate and cause a pathological effect. Various approaches to vaccines and vaccinations are described in Bernard R. Glick and Jack J. Pasternak, Molecular Biotechnology, Principles and Applications of Recombinant DNA, Second Edition, ASM Press pp. 253-276 (1998).

Vaccination is a means of inducing the body's own immune system to seek out and destroy an infecting agent before it causes a pathological response. Typically, vaccines are either live, but attenuated, infectious agents (virus or bacteria), or a killed form of the agent. A vaccine consisting of a live bacteria or virus must be non-pathogenic. Typically, a bacterial or viral culture is attenuated (weakened) by physical or chemical treatment. Although the agent is nonvirulent, it can still elicit an immune response in a subject treated with the vaccine.

An immune response is elicited by antigens, which can be either specific macromolecules or an infectious agent. These antigens are generally either proteins, polysaccharides, lipids, or glycolipids, which are recognized as "foreign" by lymphocytes known as B cells and T cells. Exposure of both types of lymphocytes to an antigen elicits a rapid cell division and differentiation response, resulting in the formation of clones of the exposed lymphocytes. B cells produce plasma cells, which in turn, produce proteins called antibodies (Ab), which selectively bind to the antigens present on the infectious agent, thus neutralizing or inactivating the pathogen (humoral immunity). In some cases, B cell response requires the assistance of CD4 helper T cells.

The specialized T cell clone that forms in response to the antigen exposure is a cytotoxic T lymphocyte (CTL), which is capable of binding to and eliminating pathogens and tissues that present the antigen (cell-mediated or cellular immunity). In some cases, an antigen presenting cell (APC) such as a dendritic cell, will envelop a pathogen or other foreign cell by endocytosis. The APC then processes the antigens from the cells and presents these antigens in the form of a histocompatibility molecule: peptide complex to the T cell receptor (TCR) on CTLs, thus stimulating an immune response.

Humoral immunity, characterized by the formation of specific antibodies, is generally most effective against acute bacterial infections and repeat infections from viruses, whereas cell-mediated immunity is most effective against viral infection, chronic intracellular bacterial infection, and fungal infection. Cellular immunity is also known to protect against certain cancers and is responsible for rejection of organ transplants.

Antibodies to antigens from prior infections remain detectable in the blood for very long periods of time, thus affording a means of determining prior exposure to a pathogen. Upon re-exposure to the same pathogen, the immune system effectively prevents reinfection by eliminating the pathogenic agent before it can proliferate and produce a pathogenic response.

The same immune response that would be elicited by a pathogen can also sometimes be produced by a non-pathogenic agent that presents the same antigen as the pathogen. In this manner, the subject can be protected against subsequent exposure to the pathogen without having previously fought off an infection.

Not all infectious agents can be readily cultured and inactivated, as is required for vaccine formation, however. Modem recombinant DNA techniques have allowed the engineering of new vaccines to seek to overcome this limitation. Infectious agents can be created that lack the pathogenic genes, thus allowing a live, nonvirulent form of the organism to be used as a vaccine. It is also possible to engineer a relatively nonpathogenic organism such as *E. coli* to present the cell surface antigens of a pathogenic carrier. The immune system of a subject vaccinated with such a transformed-carrier is "tricked" into forming antibodies to the pathogen. The antigenic proteins of a pathogenic agent can be engineered and expressed in a nonpathogenic species and the antigenic proteins can be isolated and purified to produce a "subunit vaccine." Subunit vaccines have the advantage of being stable, safe, and chemically well defined; however, their production can be cost prohibitive.

A new approach to vaccines has emerged in recent years, broadly termed genetic immunization. In this approach, a nucleic acid (polynucleotide) encoding an antigen of a pathogenic agent is operably inserted into cells in the subject to be immunized. The treated cells, preferably antigen presenting cells (APCs) such as the dendritic cells, are transformed and produce the antigenic proteins of the pathogen. These *in vivo*-produced antigens then trigger the desired immune response in the host. The genetic material utilized in such genetic vaccines can be either a DNA or RNA construct. Often the polynucleotide encoding the antigen is introduced in combination with other promoter polynucleotide sequences to enhance insertion, replication, or expression of the gene.

DNA vaccines encoding antigen genes can be introduced into the host cells of the subject by a variety of delivery systems. These delivery systems include prokaryotic and viral delivery systems. For example, one approach is to utilize a viral vector, such as vaccinia virus incorporating the new genetic material, to innoculate the host cells. Alternatively, the genetic material can be incorporated in a plasmid vector or can be delivered directly to the host cells as a "naked" polynucleotide, i.e. simply as purified DNA. In addition, the DNA can be stably transfected into attenuated bacteria such as *Salmonella typhimurium.* When a patient is orally vaccinated with the transformed *Salmonella,* the bacteria are transported to Peyer's patches in the gut (i.e., secondary lymphoid tissues), which then stimulate an immune response.

DNA vaccines provide an opportunity to immunize against disease states that are not caused by traditional pathogens, such as genetic diseases and cancer. Typically, a genetic cancer vaccine introduces into APCs a gene that encodes an antigen, and the so transformed APCs produce antigens to a specific type of tumor cell. An effective general vaccine against a number of cancer types can thus entail numerous individual vaccines for each type of cancer cell to be immunized against.

There is a continuing need for a generally effective DNA composition, such as a vaccine, for immunization against various forms of cancer. The present invention satisfies this need.

### SUMMARY OF THE INVENTION

A DNA composition effective for inhibiting tumor growth and tumor metastases comprises a DNA construct that encodes at least one epitope of fibroblast activation protein (FAP), which is expressible in immune cells of a subject to which the DNA construct has been administered. The DNA construct is incorporated in a pharmaceutically acceptable carrier. The composition can encode a single epitope of FAP, a polypeptide comprising two or more epitopes of FAP, the entire FAP protein, or any portion thereof that will elicit the desired immune response against tumor stromal cells. Preferably, the DNA construct encodes for human FAP having the amino acid residue sequence consisting of SEQ ID NO: 2 or a protein that has at least 80% sequence similarity to SEQ ID NO: 2 and which includes at least one epitope of FAP.

The DNA construct can be a naked DNA construct, preferably in the form of a plasmid. Such naked DNA constructs can be incorporated in a liposome delivery vehicle, a polymeric delivery vehicle, electroporation, gene gun, and the like, if desired. In some preferred embodiments the DNA construct is incorporated in an attenuated viral vector or an attenuated bacterial vector.

In a preferred embodiment, the DNA construct is incorporated in an attenuated bacterial vector, such as an attenuated *Salmonella typhimurium,* most preferably the doubly attenuated (*AroA ⁻*, *dam* ⁻) strain of *Salmonella typhimurium.*

Optionally, the DNA composition can also comprise a DNA construct encoding an immune effector protein, such as a cytokine. Preferred cytokines include CCL21, IL-2, and CD40LT.

The DNA compositions of the present invention can act as vaccines that target the tumor stromal fibroblast antigen FAP, which serves as a target for T cell-mediated cancer immunotherapy. Stromal fibroblasts are non-transformed cells present in the tumor environment, which support tumor growth. The approach of targeting FAP expressed by stromal fibroblast cells has several advantages over therapies directed against antigens that are solely expressed by tumor cells. First, stromal fibroblasts are genetically more stable than frequently mutating heterogeneous tumor cell populations. Accordingly, the expression of the target antigen remains more stable and serves as a more reliable target for immunotherapy. Second, antigen presentation from stromal fibroblasts to the T cell receptor complex are not impaired by downregulated MHC-antigen expression, as is frequently the case in tumor cells. Third, tumor cells often become increasingly resistant to T cell mediated killing due to defects in apoptosis signaling pathways, upregulation of antiapoptotic proteins, or immunosuppressive effects on CTLs. Additionally, targeting FAP, which is specifically overexpressed in the stromal compartment in over 90% of colon, breast and lung carcinomas allows for a therapeutic composition to treat a number of different malignancies, in contrast to therapies involving antigens that are expressed solely by specific tumor types.

In one preferred embodiment, the DNA compositions of the present invention break peripheral T cell tolerance against the FAP self-antigen by delivering its cDNA as an oral DNA composition with an attenuated bacterial delivery vector (e.g., an attenuated strain of *Salmonella typhimurium*) to antigen presenting cells in a secondary lymphoid organ, i.e., the Peyer's patches of the small intestine. In a prophylactic approach, the T cell-mediated antitumor immune response induced by vaccination with a DNA composition of the invention inhibited tumor growth in two different murine tumor models, i.e., in multi-drug resistant CT26 colon carcinoma, and multi-drug resistant D2F2 breast carcinoma. The present DNA compositions also significantly suppress the dissemination of established pulmonary metastases in a therapeutic model of CT26 colon carcinoma.

A preferred DNA composition comprises an attenuated *Salmonella* carrier, which is the doubly attenuated strain of *S*. *typhimurium* designated as RE 88 and which includes the *dam ⁻* and *AroA*⁻ mutations, available from Remedyne Corporation (Santa Barbara, CA). The attenuated *Salmonella* carrier includes DNA encoding at least one epitope of FAP, which is expressible in immune cells. The bacteria does not itself express FAP, but rather delivers the FAP DNA to immune cells (e.g., macrophages and dendritic cells), which in turn express FAP. Such compositions can prolong antitumor effects up to 10 months, and also achieve marked upregulation of T cell, NK cell and DC activation markers in murine models. Furthermore, *in vivo* depletion experiments of T cells indicated the only the involvement of CD8⁺ T cells, and no involvement of CD4⁺ T cells. The *in vitro* cytotoxic effect mediated by CD8⁺ T cells was specifically directed against target cells that overexpress the FAP antigen. For example, in a proof of concept experiment, CD8⁺ T cells from mice vaccinated with a composition of the invention induced *in vitro* cytotoxic lysis of tumor cells engineered to overexpress FAP, which indicates a specific immune response to FAP. Surprisingly, even though FAP can also be found transiently overexpressed during would healing, no impairment of wound healing caused by vaccination with the DNA compositions of the invention was observed.

The DNA compositions of the present invention also can incorporate DNA constructs that encode immune effector molecules as adjuvants for the composition. Such immune effector molecules include, for example, IL-2, an inducer of T cell proliferation; CCL21, a chemokine that chemo-attracts mature dendritic cells, and naive T cells; and CD40LT, a known inducer of dendritic cell maturation. The nucleic acids encoding the immune effector protein preferably are incorporated into a plasmid. The FAP and immune effector protein DNA constructs can be incorporated into the same plasmid or into two separate plasmids. The CTL-response induced against tumor-stromal fibroblasts can inhibit the growth of a variety of tumors, and is not specific to a particular tumor type.

The present invention also provides a method of inhibiting tumor growth and tumor metastases in a mammal comprising administering to a mammal a DNA composition of the invention in an amount sufficient to elicit an immune response against tumor-stromal cells that present the FAP antigen.

Another aspect of the present invention is an effective combination therapeutic regimen that involves administering a chemotherapeutic agent in conjunction with a DNA composition of the invention. In this method embodiment of the present invention various chemotherapeutic agents, such as doxorubicin, paclitaxel, and/or cyclophosphamide, which do not cause bone marrow suppression when administered at the maximum tolerable dose (MTD), are administered to the patient in conjunction with a DNA composition of the invention comprising a DNA construct that encodes FAP or an immunogenic portion thereof. The combination of a FAP DNA composition of the invention with such drugs offers an additional advantage, which is downregulation of collagen type I expression, and consequently, increased intratumoral uptake of various chemotherapeutic agents and other compounds. The DNA compositions of the present invention lead to increased tumor cell uptake of a variety of molecules, including, but not limited to antitumor chemotherapeutic agents. For example, the low-molecular weight dye fluorescein (376 Da), the high-molecular weight compound Evans blue albumin (68,500 Da), and the antitumor chemotherapeutic agent 5-fluorouracil (5-FU; 130 Da), all were taken up by tumor cells at much greater levels in mice treated with a DNA composition of the invention relative to mice treated with an inactive control composition. The combination of a DNA composition of the invention plus the antitumor drug doxorubicin induced complete rejection of orthotopically grown breast tumors in 50% of mice treated with the present combination therapy. Complete rejection of tumors was not observed in mice treated with a DNA composition alone or doxorubicin alone.

A preferred method provides a means for enhancing the uptake of a chemotherapeutic agent in a mammal. The method comprises administering to a mammal a DNA composition of the invention in an amount sufficient to elicit an immune response against cells in the mammal that present the FAP antigen and then administering to the mammal an effective amount of a chemotherapeutic agent.

Another preferred method embodiment is a method of inhibiting tumor growth or tumor metastases in a mammal comprising the steps of administering to a mammal a DNA composition of the invention in an amount sufficient to elicit an immune response against cells in the mammal that present the FAP antigen, and subsequently administering to the mammal an antitumor effective amount of an antitumor chemotherapeutic agent.

Preferably, the mammals treated by the methods of the present invention are humans.

In the method embodiments of the present invention, the DNA compositions can be administered enterally, such as by oral administration, or parenterally, such as by injection or intravenous infusion. Preferably, the compositions are administered orally. The compositions can be packaged in sealed containers and labeled with information useful for a clinician to effectively administer the composition.

The DNA compositions of the present invention are useful for treatment and prevention of a number of disease states. For example, a patient suffering from colorectal cancer, breast cancer, lung cancer, and the like, can benefit from immunization by the compositions of the present invention.

### BRIEF DESCRIPTION OF THE DRAWINGS

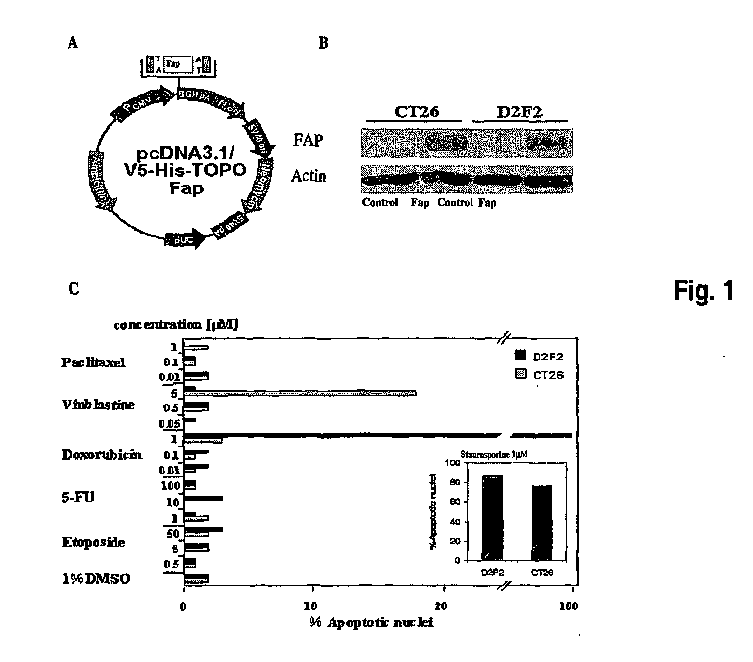

FIG. 1 is a characterization of a murine FAP DNA-construct and of chemo-resistant tumor cell lines. Panel A illustrates a plasmid cDNA encoding the entire murine FAP, inserted into the EcoRI site of the pcDNA3.1/V5-His-TOPO vector (pFAP). Panel B shows FAP protein expression was demonstrated by Western blotting after transient transfection of CT26 cells. Panel C shows CT26 colon and D2F2 breast carcinoma cells treated with various chemotherapeutic agents at the concentrations indicated. After 48 hours of incubation, nuclear apoptosis was assessed by Hoechst-33342 dye staining. Bars indicate the mean plus standard deviation of 3 assays.

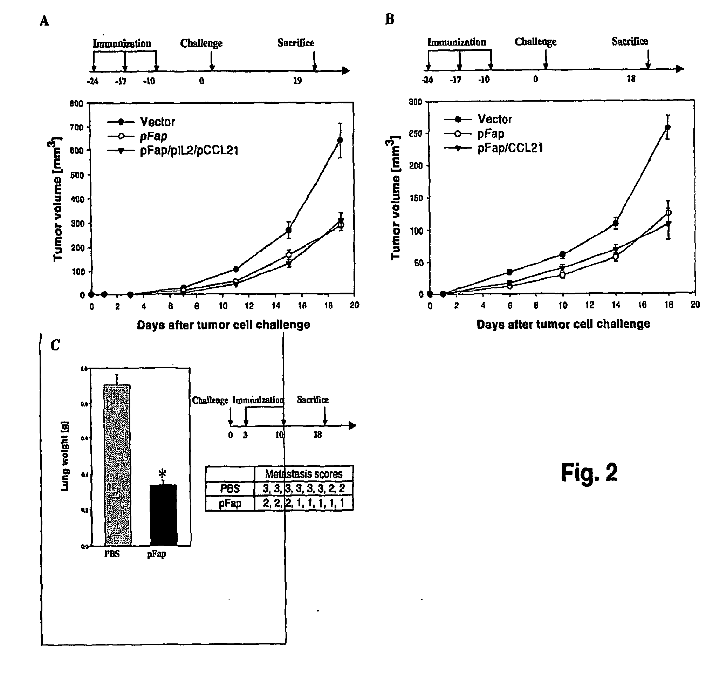

FIG. 2*a* through FIG. 2c illustrate the effect of the FAP-based DNA composition on tumor growth. Prophylactic setting: 10 days after the last of 3 vaccinations at 1-week intervals, performed as described in Materials and Methods, BALB/c mice (*n*=8) were challenged by subcutaneous (s.c.) injection with a lethal dose of 3x10⁴ CT26 cells (FIG. 2*a*) or orthotopically (o.t.) with a lethal dose of 3x10⁵ D2F2 cells (FIG. 2*b*). The average tumor growth of 8 mice is depicted, mean +/- SE, *P*<0.01. Therapeutic setting: BALB/c mice (*n*=8) were first intravenously (i.v.) injected with 10⁵ CT26 cells and then treated after 3 and 10 days once pulmonary metastases were established. After 18 days, the lungs were excised and weighed (normal lung weight was about 0.2 g), examined for metastases, and scored by a visual evaluation. The percentage of lung surface covered by fused metastases was assessed as follows: 0 = 0%, 1 = <20%, 2 = 20-50%, 3 = >50%, *P*<0.01. (FIG. 2c).

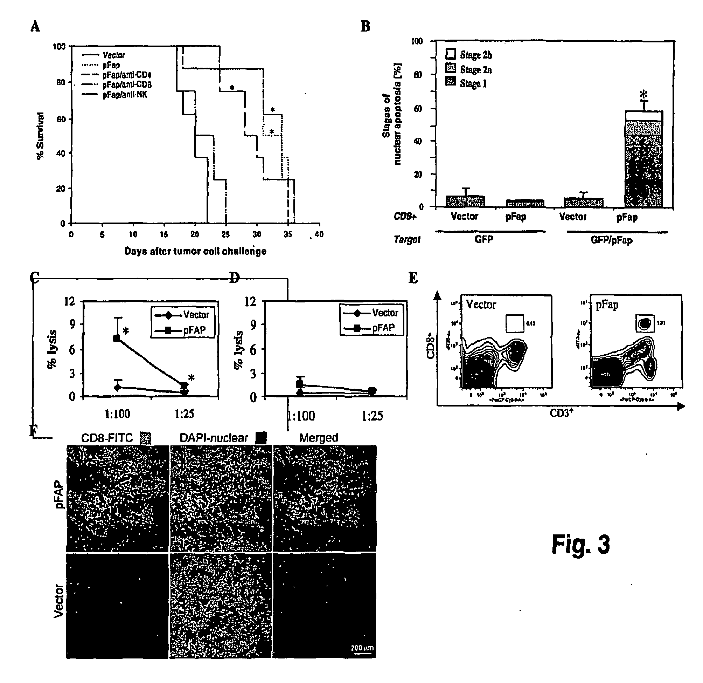

FIG. 3 illustrates cytotoxicity induced by CD8⁺ T cells. (A) Depicted is the effect on life span of antibody-mediated depletion during the effector phase in treated mice (* denotes statistical significance compared to control group, p<0.01). (B) CD8⁺ T cells were purified from spleens of treated mice, stimulated with γ-irradiated tumor target cells and then incubated for 48 hours with pGFP or pGFP/pFap-transfected CT26 cells. Nuclear apoptosis was assessed by staining with Hoechst-33342 dye as follows: Nuclear apoptosis stage 0: no apoptosis; stage 1: large-scale chromatin condensation; stage 2a: chromatin fragmentation; stage 2b: apoptotic bodies. (C, D) Splenocytes from pFap and empty vector immunized mice (*n*=3) were stimulated for 5days with pFap transfected A31 fibroblasts and then subjected to a ⁵¹Cr-release assay. (D) Effector and target cells were co-incubated with anti-MHC class I antibodies (Mean +SD, * denotes statistical significance compared to empty vector, p<0.05). (E) FACS analysis of single cell suspensions of CT26 tumors of treated mice (*n*=2) stained with anti-CD3⁺ PerCP-Cy5.5 and anti-CD8⁺ FITC antibodies. One of two experiments is depicted. (F) Representative sections of CT26 tumors of pFap and empty vector treated mice stained with anti-CD8 FITC antibodies and DAPI nuclear stain.

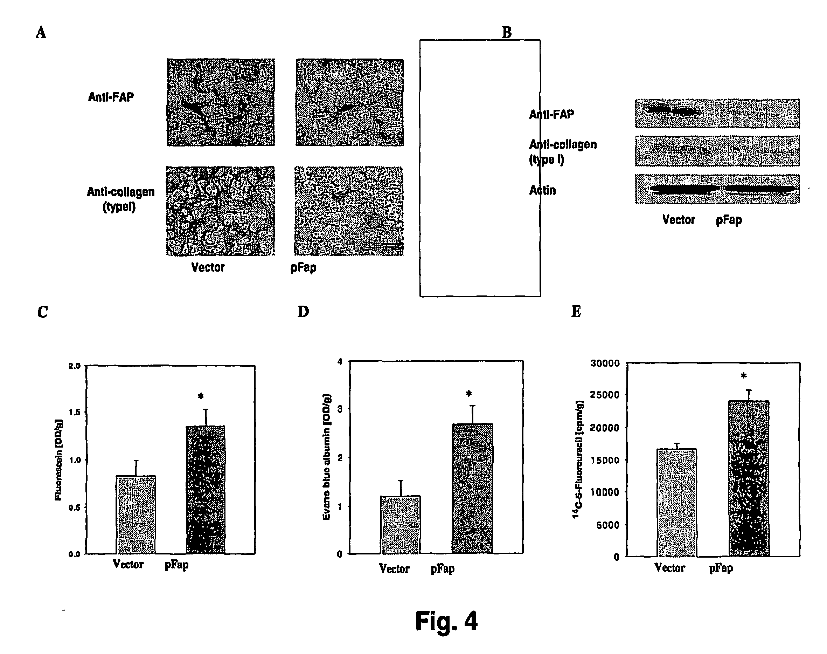

FIG. 4 demonstrates expression of FAP/collagen type I and intratumoral uptake of fluorescein/Albumin/5-FU. (A) Immunohistochemical analysis of FAP (upper panel) and collagen type I (lower panel) expression in s.c. CT26 tumors of treated mice. (B) Immunoblots of FAP and collagen type I in s.c. CT26 tumors of treated mice. (C, D, E) Bar graphs indicate means + SE of optical density or scintillation measurements of homogenates of s.c. CT26 tumors from treated BALB/c mice (n=4), after i.p. injection of fluorescein, i.v. injection of Evans blue albumin or i.v. injection of 14C-5-fluorouracil. P<0.05, P<0.01 and P<0.05, respectively.

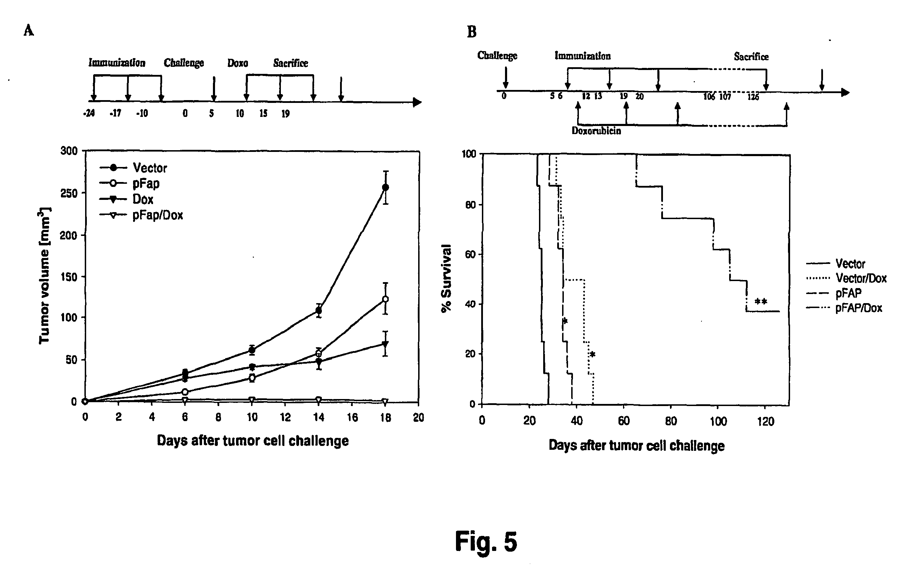

FIG. 5 illustrates the anti-metastatic effects of combined bio- and chemotherapy and side effects. (A) Prophylactic setting: 10 days after the last of 3 vaccinations at 1-week intervals with control vaccine, PBS or vaccine of the invention, BALB/c mice (*n*=8; mean ± SE) were challenged o.t. with 3x10⁵ D2F2 cells. After 5, 10 and 15 days, indicated mice were treated with doxorubicin. (B) Therapeutic setting: 5 days after i.v. injection of 105 D2F2 tumor cells BALB/c mice (*n*=8) were treated weekly with pFap vaccine or control vaccine. One day after each immunization mice were treated with doxorubicin i.v. as indicated (* depicts significant significance compared to control group, p<0.0001, ** depicts significance compared to control, control/Dox, pFap and pFap/Dox group, p<0.0001)
FIG. 6 (A) Intratumoral doxorubicin concentration: Treated BALB/c mice (*n*=4) were challenged s.c. with 5 x 10⁵ D2F2 cells and after 16 days the doxorubicin concentration in pooled tumor lysates was determined by LC-MS (representative of two experiments, mean + SD. P<0.001). (B) Circular wounds of 3 mm diameter were inflicted on the upper backs of treated mice (n=4) and the average time until complete wound closure was measured (mean + SD).
FIG. 7 shows the nucleotide sequence (SEQ ID NO: 1) of a nucleic acid encoding human FAP.
FIG. 8 illustrates the amino acid residue sequence of human FAP (SEQ ID NO: 2).
FIG. 9 shows the nucleotide sequence (SEQ ID NO: 3) of a nucleic acid encoding murine FAP.
FIG. 10 shows the amino acid residue sequence of murine FAP (SEQ ID NO: 4).
FIG. 11 shows the nucleotide sequence (SEQ ID NO: 5) of a nucleic acid encoding human IL-2.
FIG. 12 shows the amino acid residue sequence of human IL-2 (SEQ ID NO: 6).
FIG. 13 shows the nucleotide sequence (SEQ ID NO: 7) of a nucleic acid encoding murine IL-2.
FIG. 14 shows the amino acid residue sequence of murine IL-2 (SEQ ID NO: 8).
FIG. 15 shows the nucleotide sequence (SEQ ID NO: 9) of a nucleic acid encoding human CCL21.
FIG. 16 shows the amino acid residue sequence of human CCL21 (SEQ ID NO: 10).
FIG. 17 shows the nucleotide sequence (SEQ ID NO: 11) of a nucleic acid encoding murine CCL21b.
FIG. 18 shows the amino acid residue sequence of murine CCL21b (SEQ ID NO: 12).
FIG. 19 illustrates the amino acid sequence similarity between human CCL21 and murine CCL21b.
FIG. 20 shows the nucleotide sequence (SEQ ID NO: 13) of a nucleic acid encoding murine CCL21a.
FIG. 21 shows the amino acid residue sequence of murine CCL21a (SEQ ID NO: 14).
FIG. 22 shows the nucleotide sequence (SEQ ID NO: 15) of a nucleic acid encoding human CD40L
FIG. 23 shows the amino acid residue sequence of human CD40L (SEQ ID NO: 16).

### DETAILED DESCRIPTION OF PREFERRED EMBODIMENTS

The present invention provides a DNA composition effective for inhibiting tumor growth or tumor metastases, which targets the tumor stromal antigen known as fibroblast activating protein (FAP). The DNA composition comprises a DNA construct that encodes at least one epitope of FAP, which is expressible in immune cells, and which is incorporated in a pharmaceutically acceptable carrier. The DNA construct can encode a single epitope of FAP, a polypeptide comprising two or more epitopes of FAP, the entire FAP protein, or any portion thereof that will elicit the desired immune response against cells that express FAP, such as tumor stromal cells. The immune response elicited by the compositions of the invention leads to inhibition of tumor growth and tumor metastases.

The term "DNA construct" as used herein and in the appended claims refers to a DNA structure that encodes for a protein or polypeptide of interest, such as a FAP epitope, FAP protein, IL-2, CCL21, and the like. DNA constructs include any DNA that can be transcribed in target cells, including linear DNA and plasmid DNA, as well as DNA which has been incorporated into the genetic material of a cell or virus. Preferably, the DNA construct is a DNA that has been incorporated in a viral or bacterial delivery vector, e.g., an attenuated viral or bacterial vector that is non-pathogenic. When a subject is treated with a composition of the invention, the DNA encoding FAP is delivered to immune cells (e.g., macrophages and dendritic cells), which then express the FAP protein. Viral and bacterial carriers of the FAP DNA do not express FAP, themselves.

The DNA compositions of the present invention stimulate formation of CTLs that are active against cells that present the FAP antigen, such as tumor stromal cells (e.g., stromal fibroblast cells). Such tumor stromal cells are selectively targeted by CTLs that are produced in response to immunization by the DNA compositions of the invention. The compositions of the invention also can reduce tumor stromal expression of collagen type I, which in turn enhances uptake of chemotherapeutic agents.

As used herein, the term "immunity" refers to long term immunological protection against cells expressing an antigen. The term "immunization" refers to exposure to an antigen, which results in immunity to the cells expressing the antigen in the treated subject.

The term "FAP protein" refers to human FAP or a polypeptide that has at least 80 % sequence similarity to human FAP and which includes at least one epitope of human FAP. The term "FAP DNA" refers to DNA encoding human FAP or encoding a polypeptides that has at least 80 % sequence similarity to human FAP and which includes at least one epitope of human FAP.

A DNA construct useful in a DNA compositions of the present invention preferably comprises a nucleic acid that encodes a polypeptide comprising one or more epitopes of FAP, and which is operably linked to regulatory elements needed for gene expression in immune cells. Preferably, the DNA construct encodes for full length FAP protein or a polypeptide having a high degree of sequence similarity of at least 80 % therewith and which includes at lease one epitope of FAP. Useful DNA constructs preferably include regulatory elements necessary for expression of nucleotides in immune cells. Such elements include, for example, a promoter, an initiation codon, a stop codon, and a polyadenylation signal. In addition, enhancers are often required for expression of a sequence that encodes an immunogenic target protein. As is known in the art, these elements are preferably operably linked to the sequence that encodes the desired protein. Regulatory elements are preferably selected that are operable in the species to which they are to be administered. Preferably, the DNA construct is in the form of a plasmid or is incorporated into a viral or bacterial vector. The DNA construct encoding the FAP protein initially can be incorporated into a bacterial vector by transfection, using methods well known in the art. Subsequently, the transformed bacteria can be cultured to provide a ready stock of bacteria, which include FAP DNA within the genetic material of the bacteria. Cultures of such transformed bacteria provide a ready source for the DNA compositions of the present invention.

Initiation codons and stop codons are preferably included as part of a nucleotide sequence that encodes FAP in a DNA composition of the present invention. The initiation and termination codons must be in frame with the coding sequence.

Promoters and polyadenylation signals included in a composition of the present invention preferably are selected to be functional within the cells of the subject to be immunized.

Examples of promoters useful in the compositions of the present invention, especially in the production of a genetic vaccine DNA composition for humans, include but are not limited to promoters from Simian Virus 40 (SV40), Mouse Mammary Tumor Virus (MMTV) promoter, Human Immunodeficiency Virus (HIV) such as the HIV Long Terminal Repeat (LTR) promoter, Moloney virus, Cytomegalovirus (CMV) such as the CMV immediate early promoter, Epstein Barr Virus (EBV), Rous Sarcoma Virus (RSV), as well as promoters from human genes, such as human actin, human myosin, human hemoglobin, human muscle creatine, and human metalothionein.

Examples of polyadenylation signals useful in the DNA compositions of the present invention, especially in the production of a DNA composition for humans, include but are not limited to SV40 polyadenylation signals and LTR polyadenylation signals.

In addition to the regulatory elements required for DNA expression, other elements can also be included in the DNA molecule. Such additional elements include enhancers. The enhancer can be, for example, human actin, human myosin, human hemoglobin, human muscle creatine and viral enhancers such as those from CMV, RSV and EBV.

Regulatory sequences and codons are generally species dependent, so in order to maximize protein production, the regulatory sequences and codons are preferably selected to be effective in the species to be immunized. One having ordinary skill in the art can produce DNA constructs that are functional in a given subject species.

DNA constructs useful in the present compositions can be "naked" DNA, as defined in Restifo et al. Gene Therapy 2000; 7:89-92, the relevant disclosure of which is incorporated by reference. Preferably, the DNA construct is in the form of a plasmid or DNA that is incorporated into the genetic material of an attenuated virus or attenuated bacterium. Useful delivery vehicles or carriers include biodegradable microcapsules, immuno-stimulating complexes (ISCOMs), and liposomes for naked DNA constructs, and various physiologically acceptable buffers for genetically engineered attenuated live viruses or bacteria.

Examples of suitable attenuated live bacterial vectors that can be transformed to incorporate a FAP DNA construct include *Salmonella typhimurium, Salmonella typhi, Shigella* species, *Bacillus* species, *Lactobacillus* species, *Bacille Calmette-Guerin (BCG), Escherichia coli, Vibrio cholerae, Campylobacter* species, *Listeria* species, or any other suitable bacterial vector, as is known in the art. Preferably, the vector is an attenuated live *Salmonella typhimurium* vector, particularly when the composition is intended for oral administration. Preferred attenuated live *Salmonella typhimurium* include *AroA ⁻* strains such as SL7207, or doubly attenuated *AroA ⁻, dam ⁻* strains, such as RE88. The doubly attenuated *AroA ⁻, dam ⁻ Salmonella typhimurium* is a particularly preferred vector.

Methods of transforming live bacterial vectors with an exogenous DNA construct are well described in the art. See, for example, Joseph Sambrook and David W. Russell, Molecular Cloning, A Laboratory Manual, 3rd Ed., Cold Spring Harbor Laboratory Press, Cold Spring Harbor, New York (2001) (Sambrook and Russell). After transformation, the exogenous genetic material is incorporated into the genetic material of the bacterium, so that as the bacteria reproduce, the exogenous DNA is replicated along with the native DNA of the organism. Thus, once the bacterium has been transformed, the normal reproductive processes of the organism provides a ready supply of the exogenous DNA.

Preferred viral vectors include Bacteriophages, Herpes virus, Adenovirus, Adeno-associated virus, Sindbis virus, Polio virus, Vaccinia virus, and Avipox. Methods of transforming viral vector with an exogenous DNA construct are also well described in the art. *See* Sambrook and Russell, above.

Useful liposome carrier vehicles are unilamellar or multilamellar vesicles, having a membrane portion formed of lipophilic material and an interior aqueous portion. The aqueous portion is used in the present invention to contain the polynucleotide material to be delivered to the target cell. It is generally preferred that the liposome forming materials have a cationic group, such as a quaternary ammonium group, and one or more lipophilic groups, such as saturated or unsaturated alkyl groups having about 6 to about 30 carbon atoms. One group of suitable materials is described in European Patent Publication No. 0187702, and further discussed in U.S. Patent No. 6,228,844 to Wolff et al.*,* the relevant disclosures of which are incorporated by reference. Many other suitable liposome-forming cationic lipid compounds are described in the literature. See, e.g., L. Stamatatos et al., Biochemistry 1988; 27:3917-3925; and H. Eibl et al., Biophysical Chemistry 1979; 10:261-271. Alternatively, a microsphere such as a polylactide-coglycolide biodegradable microsphere can be utilized. A nucleic acid construct is encapsulated or otherwise complexed with the liposome or microsphere for delivery of the nucleic acid to a tissue, as is known in the art.

Other useful carrier vehicles include polymeric microspheres comprising biodegradable poly(ortho ester) materials, as described by Wang et al., Nat. Mater., 2004; 3(3):190-6. Epub 2004 Feb. 15, the relevant disclosures of which are incorporated herein by reference.

Preferably, the compositions for the present invention comprise DNA constructs that encode human FAP or a functional homolog thereof. Functional homologs of FAP preferably have at least about 80% amino acid residue sequence similarity to human FAP more preferably, at least about 90%, most preferably at least about 95% sequence similarity to human FAP.

GenBank is a genetic sequence database of the National Institutes of Health (NIH), which is an annotated collection of all publicly available DNA sequences. GenBank is part of the International Nucleotide Sequence Database Collaboration, a combined effort of the DNA DataBank of Japan (DDBJ), the European Molecular Biology Laboratory (EMBL), and GenBank at the National Center for Biotechnology Information.

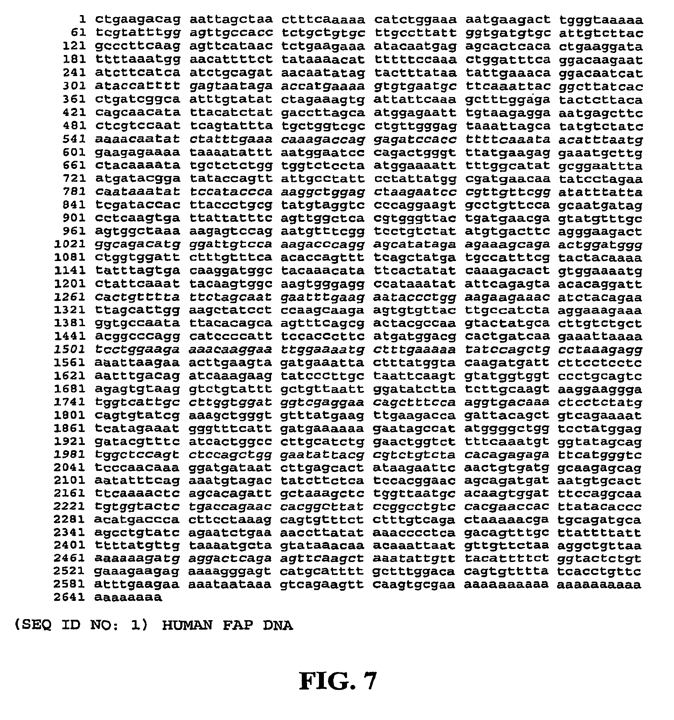

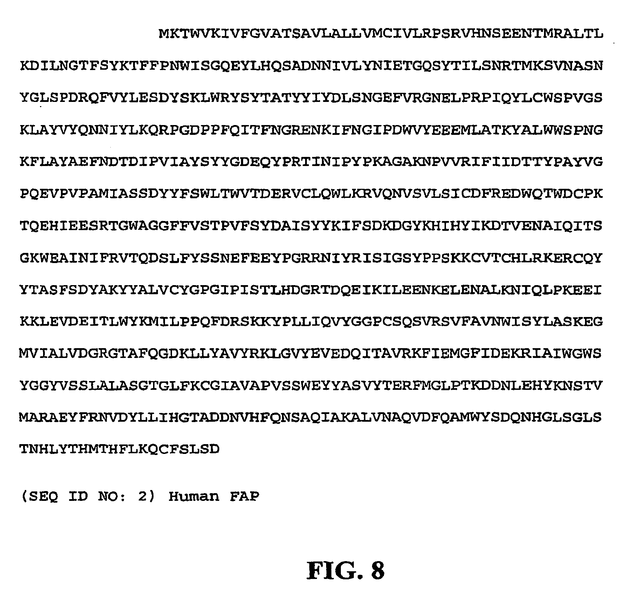

The nucleic acid sequence of a cDNA encoding human FAP, SEQ ID NO: 1 (FIG. 7) has been published in GenBank, Accession No. BC026250, the disclosure of which is incorporated herein by reference. The corresponding amino acid residue sequence of human FAP is SEQ ID NO: 2 (FIG. 8).

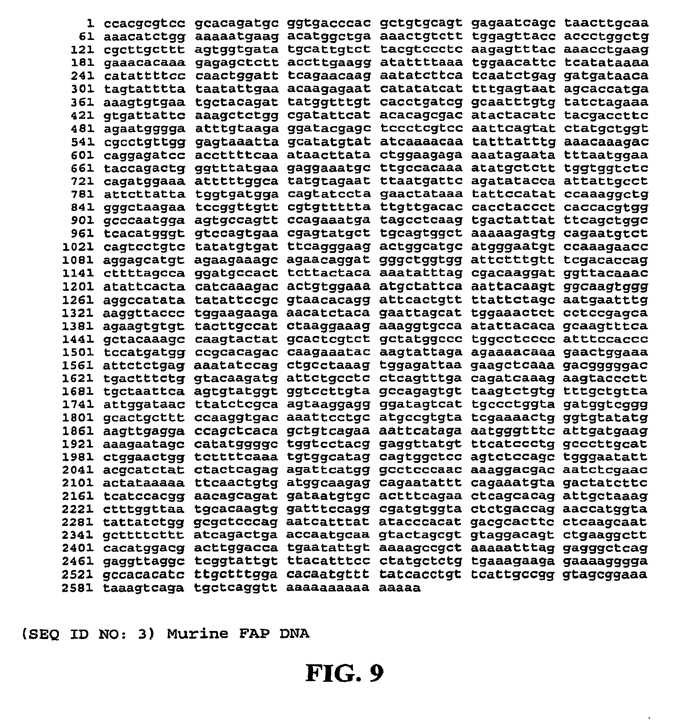

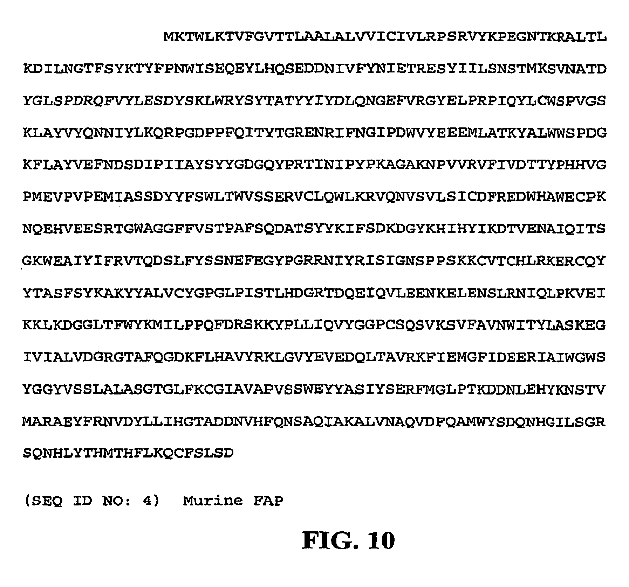

The nucleic acid sequence of a DNA encoding murine FAP, SEQ ID NO: 3 (FIG. 9) has been published in GenBank, Accession No. BC019190, the disclosure of which is incorporated herein by reference. The corresponding amino acid residue sequence of murine FAP is SEQ ID NO: 4 (FIG. 10).

Due to the inherent degeneracy of the genetic code, other DNA sequences that encode the amino acid sequence to human FAP, can be used in the practice of the invention. Such DNA sequences include those which are capable of hybridizing to human FAP as well.

DNA sequences that encode human FAP, which can be used in accordance with the invention include nucleic acids having deletions, additions or substitutions of different nucleotide residues for those in SEQ ID NO: 1, which result in a sequence that encodes the same FAP gene product. DNA molecules encoding for functionally equivalent homologs of human FAP can also be used in the DNA compositions of the present invention.

The gene product encoded by nucleic acid may also contain deletions, additions or substitutions of amino acid residues within the FAP amino acid residue sequence, which results in a silent change, thus producing a functionally equivalent FAP. Such amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine. As used herein, a functionally equivalent FAP refers to protein that includes one or more epitopes, which when recognized by T cells, allow these same T cells to recognize FAP epitopes displayed on FAP-expressing cells. In preferred embodiments, a functionally equivalent FAP has an amino acid residue sequence that has at least about 80% sequence similarity to the amino acid residue sequence of human FAP (SEQ ID NO: 2), e.g., at least about 90% sequence similarity or at least about 95% sequence similarity to human FAP.

The DNA constructs encoding FAP can be engineered in order to alter the FAP coding sequence (relative to the native FAP cDNA, SEQ ID NO: 1) for a variety of ends including, but not limited to, alterations that modify processing and expression of the FAP gene product. For example, mutations may be introduced in the DNA using techniques that are well known in the art, e.g., site-directed mutagenesis, to insert new restriction sites, to alter glycosylation patterns, phosphorylation, etc.

In one preferred embodiment, the DNA composition of the invention comprises a DNA construct encoding FAP and a DNA construct operably encoding at least one immune effector protein, both of which are expressible in immune cells. As used herein and in the appended claims the phrase "immune effector protein" means a protein that is involved in regulation of an immune system pathway. Preferably, the immune effector protein is a cytokine.

Cytokines are proteins and polypeptides produced by cells that can affect the behavior of other cells, such as cell proliferation, cell differentiation, regulation of immune responses, hematopoiesis, and inflammatory responses. Cytokines have been classified into a number of families, including chemokines, hematopoietins, immunoglobulins, tumor necrosis factors, and a variety of unassigned molecules. *See generally* Oxford Dictionary of Biochemistry and Molecular Biology, Revised Edition, Oxford University Press, 2000; and C. A. Janeway, P. Travers, M. Walport and M. Schlomchik, Immunobiology, Fifth Edition, Garland Publishing, 2001 (hereinafter "Janeway and Travers"). A concise classification of cytokines is presented in Janeway and Travers, Appendix III, pages 677-679, the relevant disclosures of which are incorporated herein by reference.

Hematopoietins include, for example erythropoietin, interleukin-2 (IL-2, a 133 amino acid protein produced by T cells and involved in T cell proliferation), IL-3, IL-4, IL-5, IL-6, IL-7, IL-9, IL-11, IL-13, IL-15 (a 114 amino acid IL-2-like protein, which stimulates the growth of intestinal epithelium, T cells, and NK cells), granulocyte colony-stimulating factor (G-CSF), granulocyte-macrophage colony-stimulating factor (GM-CSF), oncostatin M (OSM), and leukemia inhibitory factor (LIF).

Interferons include, for example, IFN-α, IFN-β, and IFN-γ (a 143 amino acid homodimeric protein produced by T cells and NK cells, which is involved in macrophage activation, increased expression of MHC molecules and antigen processing components, IG class switching, and suppression of T_{H}2).

Immunoglobulins include, for example, B7.1 (CD80), and B7.2 (CD86), both of which co-stimulate T cell responses.

The tumor necrosis factor (TNF) family includes, for example, TNF-α, TNF-β (lymphotoxin), lymphotoxin-β (LT-β), CD40 ligands, Fas ligand, CD27 ligand, CD30 ligand, 4-1BB ligand, Trail, and OPG ligand.

The biological roles of CD40 ligand (CD40L), particularly its interaction with CD40 expressed on antigen presenting cells during costimulation of T cell activation, are well known in the art. CD40 is a 48 kDa glycoprotein expressed on the surface of all mature B cells, most mature B-cell malignancies, and some early B-cell acute lymphocytic leukemias, but it is not expressed on plasma cells, Clark, Tissue Antigens 1990, 35:33-36. CD40L, a type II membrane protein of about 35 kDa, is expressed on the surface of T cells upon antigen recognition. Members of the TNF family are biologically most active when expressed as homotrimers. CD40L is no exception in this regard and can be expressed as a homotrimer (CD40LT) by modification of a 33 amino acid leucine zipper motif fused to the N-terminus of the entire extracellular domain of this ligand. CD40LT DNA has been reported by Gurunathan et al. J. Immunol. 1998, 161:4563, to enhance cellular immune responses such as induction of IFN-γ and cytolytic T cell activity when mice were treated with DNA encoding the highly immunogenic model antigen β-galactosidase.

CD40LT is an important factor in the activation of T cells necessary to induce an effective protective immunity against tumor self-antigens. Once antigen loaded MHC class I complexes are taken up by dendritic cells (DCs) and presented to naive T cells, the first antigen signal is delivered via T cell receptors (TCR), followed by upregulation of CD40LT. On the T cell surface, CD40LT then induces costimulatory activity on DCs via CD40-CD40LT interactions. Thus primed, these APCs can express costimulatory molecules B7.1 (CD80) and B7.2 (CD86), which sends a second costimulatory signal to T cells via interaction with CD28, an event that leads to full activation of T cells to concurrently produce pro-inflammatory cytokines INF-γ and IL12, and to perform effector functions.

Various cytokines that are not assigned to a particular family include, for example, tumor growth factor-β (TGF-β), IL-1α, IL-1β, IL-1 RA, IL-10, IL-12 (natural killer cell stimulatory factor; a heterodimer having a 197 amino acid chain and a 306 amino acid chain, which is involved in NK cell activation and induction of T cell differentiation to T_{H}1-like cells), macrophage inhibitory factor (MIF), IL-16, IL-17 (a cytokine production-inducing factor, which induces cytokine production in epithelia, endothelia, and fibroblasts), and IL-18.

Chemokines are a family of cytokines that are relatively small chemoattractant proteins and polypeptides, which stimulate the migration and activation of various cells, such as leucocyte migration (e.g., phagocytes and lymphocytes). Chemokines play a role in inflammation and other immune responses. Chemokines have been classified into a number of families, including the C chemokines, CC chemokines, CXC chemokines, and CX₃C chemokines. The names refer to the number and spacing of cysteine (C) residues in the molecules; C chemokines having one cysteine, CC chemokines having two contiguous cysteines, CXC having two cysteines separated by a single amino acid residue, and CX₃C chemokines having two cysteines separated by three amino acid residues. Chemokines interact with a number of chemokine receptors present on cell surfaces. See Janeway and Travers, Appendix IV, page 680, which is incorporated herein by reference.

In addition, chemokines can have immunomodulating activity and have been implicated in immune responses to cancer. For example, murine 6Ckine/SLC, the mouse analog of the human secondary lymphoid tissue chemokine (SLC), now commonly referred to as CCL21, has been reported to induce an antitumor response in a C26 colon carcinoma tumor cell line. See Vicari et al. J. Immunol. 2000; 165(4):1992-2000. Human CCL21 and its murine counterpart, 6Ckine/SLC, are classified as CC chemokines, which interact with the CCR7 chemokine receptor. Murine 6Ckine/SLC (muCCL21) is also reported by Vicari *et al.* to be a ligand for the CXCR3 chemokine receptor. Human CCL21, murine muCCL21, and a variety of other chemokines are implicated in the regulation of various immune system cells such as dendritic cells, T cells, and natural killer (NK) cells.

Mig and IP-10 are CXC chemokines that interact with the CXCR3 receptor, which is associated with activated T cells. Lymphotactin is a C chemokine, which interacts with the XCR1 receptor associated with T cells and NK cells. Fractalkine is a CX₃C chemokine, which interact with the CX₃CR1 receptor that is associated with T cells, monocytes and neutrophils.

Particularly preferred immune effector proteins to be encoded by the DNA compositions of the present invention include cytokines IL-2 (a hematopoietin), CCL21 (a chemokine), as well as CD40 ligands such as CD40 ligand trimer (CD40LT), a TNF family cytokine.

DNA and protein sequences for human IL-2 have been published in GenBank, Accession No. BC070338, the disclosures of which are incorporated herein by reference. The DNA and protein sequences of murine IL-2 have been in GenBank, Accession No. NM 008366, the disclosures of which are incorporated herein by reference.

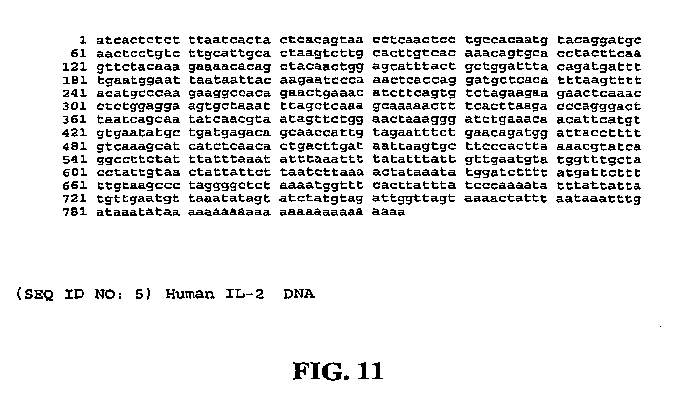

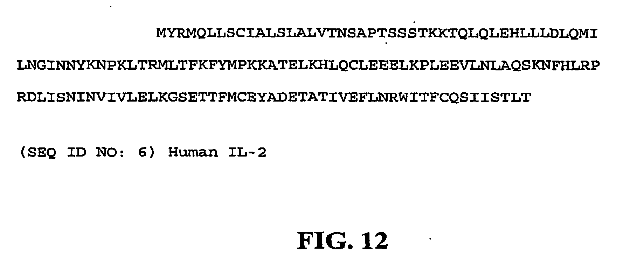

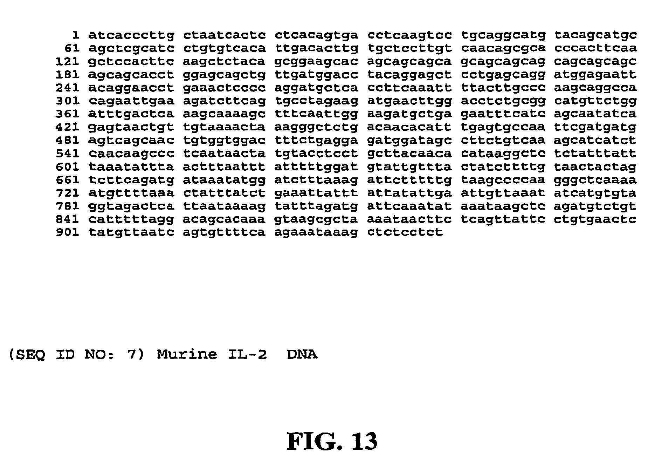

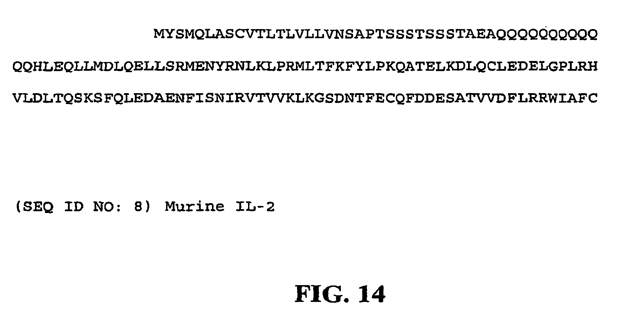

The nucleic acid sequence encoding human IL-2 is presented in FIG. 11 (SEQ ID NO: 5), and its corresponding amino acid residue sequence (SEQ ID NO: 6) is provided in FIG. 12. The nucleic acid sequence encoding murine IL-2 is presented in FIG. 13 (SEQ ID NO: 7), and its corresponding amino sequence (SEQ ID NO: 8) is provided in FIG. 14.

DNA and protein sequences for human CCL21 have been published in GenBank, Accession No. AB002409, the disclosures of which are incorporated herein by reference. The DNA and protein sequences of the murine CCL21 a variant have been published in GenBank, Accession No. NM011335, the disclosures of which are incorporated herein by reference. The DNA and protein sequences of the murine CCL21b variant have been published in GenBank, Accession No. NM011124, the disclosures of which are incorporated herein by reference.

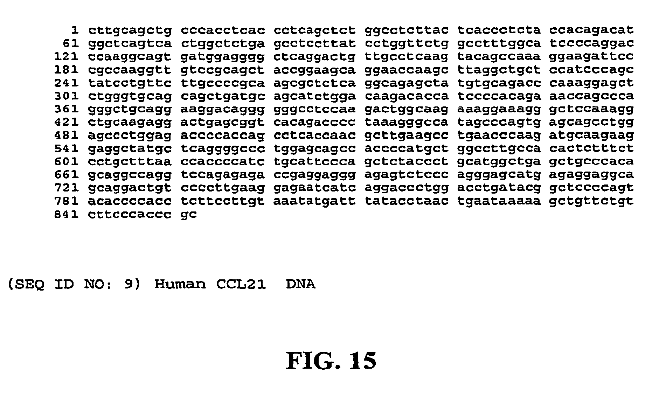

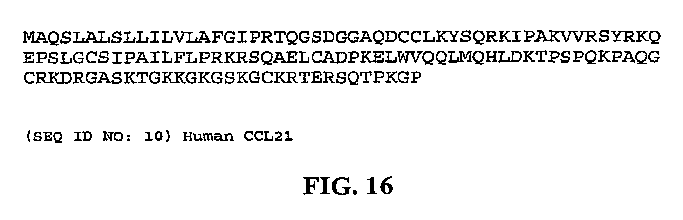

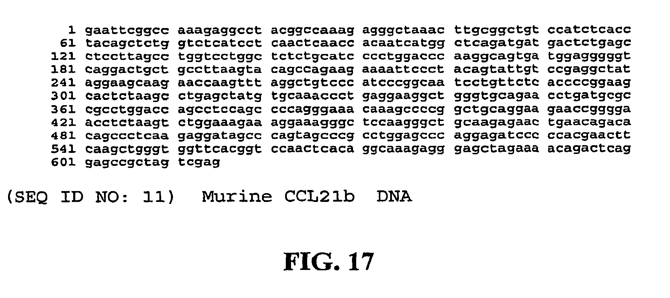

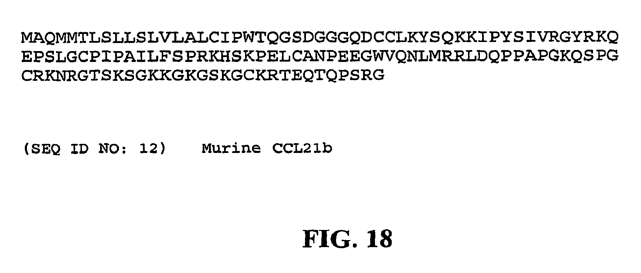

The nucleic acid sequence encoding human CCL21 is presented in FIG. 15 (SEQ ID NO: 9), and its corresponding amino acid residue sequence (SEQ ID NO: 10) is provided in FIG. 16. The nucleic acid sequence encoding murine CCL21 (CCL21 b variant) is presented in FIG. 17 (SEQ ID NO: 11), and its corresponding amino sequence (SEQ ID NO: 12) is provided in FIG. 18.

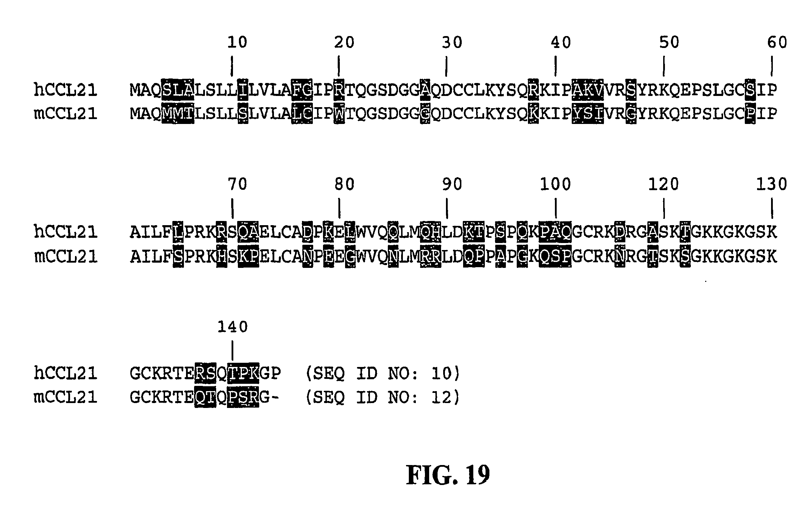

The protein sequence similarity between human CCL21 and its murine counterpart (murine CCL21b) is illustrated in FIG. 19. There is about 73% amino acid residue sequence identity between human CCL21 (SEQ ID NO: 10) and murine CCL21b (SEQ ID NO: 12).

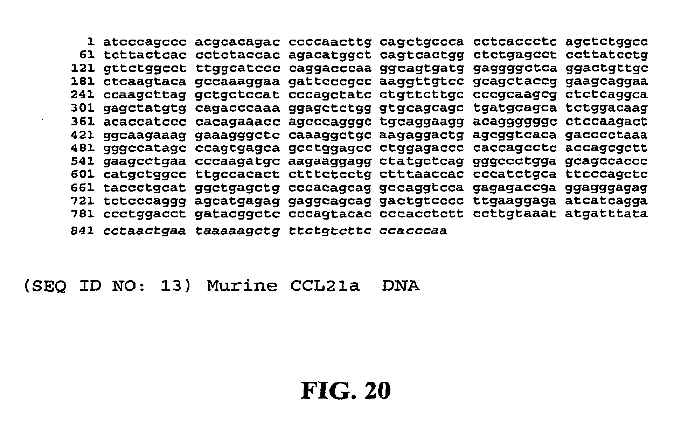

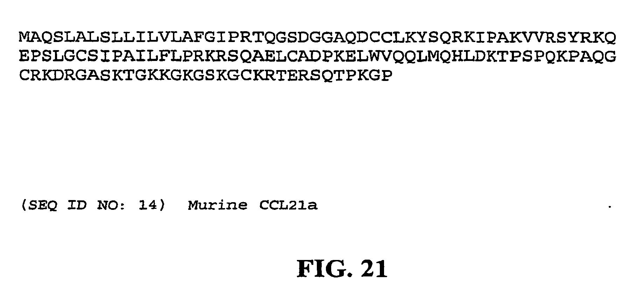

The nucleic acid sequence encoding the CCL21a variant of murine CCL21 is presented in FIG. 20 (SEQ ID NO: 13), and its corresponding amino sequence (SEQ ID NO: 14) is provided in FIG. 21.

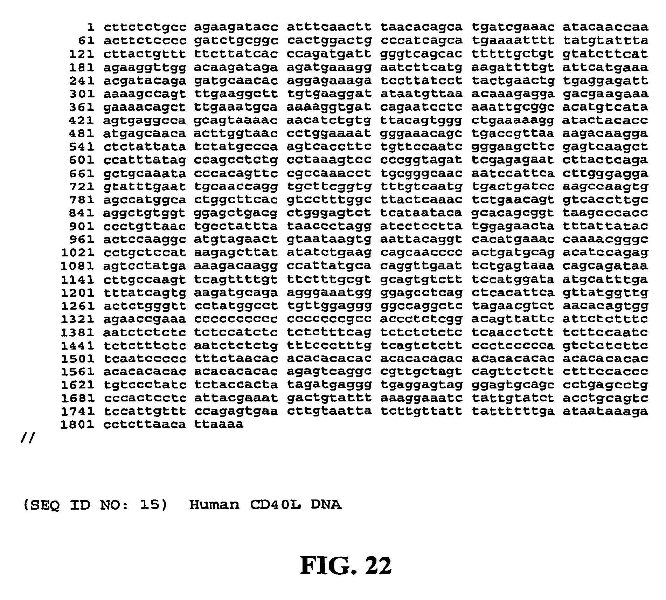

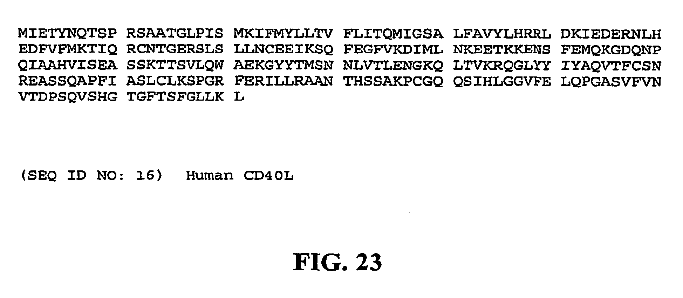

Human CD40 ligand (CD40L) is a 261 amino acid protein, which exists as a trimer (CD40LT) in its most active form. The DNA sequence encoding human CD40L (also known as CD154) has been published in GenBank, Accession No. NM 000074, the disclosure of which is incorporated herein by reference (FIG. 22, SEQ ID NO: 15). The corresponding protein sequence of CD40L is shown in FIG. 23 (SEQ ID NO: 16).

The method aspects of the present invention involve administering to a mammal a DNA composition comprising a DNA construct encoding FAP, which is expressible in immune cells of the mammal. Preferably, the mammal is a human. The composition can be administered orally, intramuscularly, intranasally, intraperitoneally, subcutaneously, intradermally, or topically, depending upon the particular dosage form in which the composition is prepared. Preferably the composition is prepared in an orally administrable dosage form, such as a solution, suspension, emulsion, capsule, tablet, and the like.

A DNA composition of the invention can be utilized to provide long term inhibition of tumor growth and/or tumor metastases in a patient treated with the composition. In a preferred embodiment, the DNA composition is administered in conjunction with an antitumor chemotherapeutic agent. The DNA composition can be administered together with the chemotherapeutic agent in a combined dosage form, or the composition and chemotherapeutic agent can be administered in separate dosage forms and at separate dosage intervals tailored to the pharmacology of the chemotherapeutic agent being administered.

Chemotherapeutic agents useful in combination with the DNA compositions of the present invention include antitumor agents such as doxorubicin, paclitaxol, a cyclophosphamide, etoposide, 5-fluorouracil, methotrexate, and the like.

DNA compositions of the present invention preferably are formulated with pharmaceutically acceptable carriers or excipients such as water, saline, dextrose, glycerol, and the like, and combinations thereof to aid in formulation and administration of the composition. The compositions can also contain auxiliary substances such as wetting agents, emulsifying agents, buffers, and other auxiliary substances that are well known in the pharmaceutical arts.

The compositions of the present invention are preferably administered orally to a mammal, such as a human, as a solution or suspension in a pharmaceutically acceptable carrier, at a DNA concentration in the range of about 1 to about 10 micrograms per milliliter, based on the weight of the DNA that encodes FAP. A particularly preferred dosage form for a DNA composition of the invention is a suspension of attenuated FAP-transfected bacteria in a suitable buffer solution, which can be formulated for oral administration. The appropriate dosage of the composition will depend upon the subject to be treated, on the activity of the composition, and in part upon the judgment of the medical practitioner administering or requesting administration of the composition.

The dosage to be administered to the mammal and the schedule of administration if more than one administration is to be used, will vary from mammal to mammal and upon the dosage form. Effective dosage amounts and administration schedules can be determined empirically through clinical dose-response studies, as is well known in the art. The dosage and dosage schedule is selected to provide a sufficient amount of FAP expression in immune cells to elicit an immune response in the mammal against cells that present the FAP antigen. Preferably, the dosage of the composition administered to the subject mammal elicits expression of a sufficient amount of FAP antigen in immune cells of the mammal to sustain an immune response against FAP-presenting cells that will continue over a period of at least a month, e.g., at least 6 months or at least about one year.

The compositions of the present invention can be packaged in suitably sterilized containers such as ampules, bottles, or vials, either in multi-dose or in unit dosage forms. The containers are preferably hermetically sealed after being filled with a DNA composition of the invention. Preferably, the compositions are packaged in a container having a label affixed thereto, which label identifies the composition, and bears a notice in a form prescribed by a government agency such as the United States Food and Drug Administration reflecting approval of the composition under appropriate laws, dosage information, and the like. The label preferably contains information about the composition that is useful to an health care professional administering the composition to a patient. The package also preferably contains printed informational materials relating to the administration of the composition, instructions, indications, and any necessary required warnings.

The following examples are provided to further illustrate the features and embodiments of the present invention, and are not meant to be limiting.

### Materials, Methods and Examples.

**Animals, bacterial strains, and cell lines.** Female BALB/c mice, 6-8 weeks of age, were obtained from the Scripps Research Institute (TSRI) Rodent Breeding Facility. All animal experiments were performed according to the National Institutes of Health Guide for the Care and Use of Laboratory Animals and approved by the TSRI Animal Care Committee. The double attenuated *Salmonella typhimurium* strain RE88 (*AroA ⁻ dam ⁻*) was provided by Remedyne Corporation (Santa Barbara, California). Murine D2F2 breast carcinoma cells were obtained from Dr. Wei-Zen Wei, Karmanos Cancer Center, Detroit, MI, and CT26 colon carcinoma cells were obtained from ATCC (Manassas, Virginia) and then cultivated over several months to obtain chemoresistant subclones.

Murine CT26 and D2F2 carcinoma cells were cultured in the presence of either 1% DMSO, etoposide, 5-fluorouracil, doxorubicin, vinblastine or paclitaxel (Sigma, St. Louis, Missouri) at the concentrations indicated. After 48 hours, apoptotic nuclei were counted after incubation with the DNA-specific Hoechst-33342 dye (2 µM; Molecular Probes, Eugene, Oregon).

Two clones of CT26 colon cancer and D2F2 breast cancer cells, respectively, have acquired chemoresistance (see FIG. 1, Panel C). Upon addition of five different apoptosis-inducing chemotherapeutic agents to the CT26 colon carcinoma clone (FIG. 1, Panel C), vinblastine alone was able to induce a slight apoptotic effect, however only at the very high concentration of 5 µM. The original parent CT26 cell line (ATCC # CLR-2326) had an IC₅₀ for 5-fluorouracil (5-FU) of about 1.85 µM, whereas 5-FU is not able to induce nuclear apoptosis even at a concentration as high as 100 µM in the resistant CT26 clone. In the resistant D2F2 breast carcinoma cells, only doxorubicin at the highest concentration of 1 µM was able to induce nuclear apoptosis as determined by Hoechst-33342 dye staining. These results demonstrate that both of these tumor cell lines are multi-drug resistant.

**Statistical analysis.** The statistical significance of differential findings between experimental groups and controls was determined by Student's t-test. Significance of metastasis scores were determined by the Mann Whitney U-test. Significance of survival data was determined by the log-rank test. Findings were regarded significant, if P values were <0.05.

### EXAMPLE 1. Preparation of DNA Composition 1 Encoding Murine FAP.

A cDNA (SEQ ID NO: 3, FIG. 9; provided by Dr. J. D. Cheng) encoding murine FAP (SEQ ID NO: 4, FIG. 10), was subcloned into the EcoRI restriction site of the pcDNA3.1/V5-His-TOPO vector (Invitrogen, San Diego, California), to afford the eukaryotic expression vector pcDNA3.1-FAP (pFAP) (see FIG. 1, Panel A). The correct expression of the 95 kDa FAP protein from the vector was demonstrated by Western-blotting of cell lysates from transiently transfected CT26 colon carcinoma cells, which do not express FAP by themselves (FIG. 1, Panel B). This was also the case for transiently transfected D2F2 breast carcinoma cells. For transient transfections, CT26 and D2F2 cells were electroporated as described previously (See Loeffer et al., FASEB, J. 2001, 15: 758-767, incorporated herein by reference). Protein expression of FAP was demonstrated by Western blotting with a polyclonal rabbit anti-murine FAP antibody (provided by Dr. J. D. Cheng).

DNA composition 1 was prepared as follows: the pcDNA3.1-FAP vector (about 2 µg of pDNA) was electroporated into freshly prepared doubly attenuated *Salmonella typhimurium* (strain RE88), utilizing a Bio-Rad Pulser at 2 kV, 960 µF, and 200 Ohm according to the manufacturer's recommended procedures. Attenuated *Salmonella typhimurium* containing the vector were selected on ampicillin-containing plates. Colonies were picked the next day and cultured overnight in Luria-Bertani (LB) broth (10 grams tryptone, 5 grams yeast extract, and 10 grams sodium chloride in 1 L of deionized water; EM Science, Gibbstown, NJ) with ampicillin added. The bacteria were isolated and washed in phosphate buffered saline (PBS). The washed bacteria were then suspended in PBS medium at a concentration of about 10⁹ recombinant *Salmonella* per milliliter of PBS, to form a solution of DNA Composition 1 containing a DNA construct that operably encodes for murine FAP, incorporated in an attenuated *Salmonella typhimurium* (*AroA ⁻ dam ⁻*) vector. The composition was stored in sealed ampules until used. A "control vaccine" consisting of *Salmonella* transformed with the pcDNA3.1 vector alone (no FAP DNA) was also prepared according to the same procedure. The plasmid DNA was stored at about -80 °C before transforming the *Salmonella.*

After constructing the eukaryotic expression vector pcDNA3.1/V5-His-TOPO-Fap (pFap) as described above (FIG. 1, Panel A), the correct expression of the 88 kDa FAP protein was demonstrated by Western-blotting of cell lysates from both, transiently transfected CT26 colon carcinoma and D2F2 breast carcinoma cells, which themselves do not express FAP (FIG. 1, Panel B).

### EXAMPLE 2. Preparation of DNA Composition 2 Encoding Murine FAP , Murine IL-2, and Murine CCL21.

cDNAs encoding murine IL-2 (DNA SEQ ID NO: 5), from ATCC, Accession # 39892, Manassas, Virginia, and murine CCL21 a (DNA SEQ ID NO: 7) from Invitrogen, San Diego, California, were subcloned into the murine pFAP vector of Example 1, by the same general procedure described in Example 1. RE88 *Salmonella typhimurium* were transformed with the resulting vector (pFAP/IL-2/CCL21) by the procedure described in Example 1 to provide a solution of DNA Composition 2 containing a DNA construct that operably encodes murine FAP, murine IL-2, and murine CCL21a, incorporated in an attenuated *Salmonella typhimurium* vector.

### EXAMPLE 3. Evaluation of DNA compositions of the Invention in Murine Models of Colon and Breast Cancer.

**Oral immunization, tumor cell challenge and treatment with doxorubicin.** For experiments in a prophylactic setting, BALB/c mice (*n*=8) were treated three times at about 1-week intervals by oral gavage with about 100 µl PBS containing about 10⁹ *S. typhimurium* (*AroA ⁻ dam ⁻*) transformed with plasmid vectors encoding murine FAP (DNA Composition 1 of Example 1), murine FAP, IL-2 and CCL21 (DNA Composition 2 of Example 2), or control vaccine of Example I, by methods described in Niethammer et al., Nat. Med., 2002, 8: 1369-1375. Animals were challenged about 10 days later by subcutaneous (s.c.) injection of about 3x10⁴ CT26 colon carcinoma cells in the left front flank or by orthotopic (o.t.) injection of about 3x10⁵ D2F2 breast cancer cells in the left, second lowest mammary fat pad.

Tumor volume (in mm³) was calculated by measuring the tumor in 2 dimensions (i.e., length and width, in millimeters) and calculating the volume as one half the length times the square of the width. In the therapeutic setting, the initial tumor cell inoculation was by intravenous (i.v.) injection of about 1x10⁵ CT26 colon carcinoma cells followed about 3 and 10 days later by oral vaccinations with control vaccine or active DNA compositions. After about 18 days, the lungs were weighed (normal lung weight was about 0.2 g) and pulmonary tumor metastases were examined and scored by visual evaluation assessing the percentage of lung surface covered by fused metastases as follows: score of 0 for 0% coverage, score of 1 for less than about 20% coverage, score of 2 for about 20-50% coverage, score of 3 for greater than about 50% of lung surface covered. Treatment with doxorubicin was performed in groups of mice with about 10 mg/kg doxorubicin (Sigma) administered i.v., 5, 10 and 15 days after tumor challenge.

For the depletion of CD4⁺ and CD8⁺ T cells or NK cell subpopulations, antibodies (500 µg) directed against either CD4 (clone GK 1.5) or CD8 (clone 2.43), both from National Cell Culture Center (Minneapolis, Minnesota), or anti-asialo GM1 antibody (Wako BioProducts, Richmont, Virginia) were injected i.p. every 7 days starting one day before tumor cell challenge.

**Cytotoxicity of CD8⁺ T cells.** About 10 days after the last of three treatments at 1 week intervals, splenocytes from the various treated groups of BALB/c mice (*n*=4) were collected. Using CD8a-micro beads (Miltenyi Biotech, Bergisch Gladbach, Germany), CD8⁺ cells were purified according to the manufacturer's protocol. These cells were then stimulated in a 5-day co-culture with γ-irradiated (1000 Gy, 45 minutes) CT26 cells were transiently transfected with either pcDNA3.1/Zeo (empty vector of Example 1) or pcDNA3.1/Zeo-FAP (pFAP vector of Example 1). Thereafter, the CD8⁺ T cells were co-cultivated with CT26 carcinoma cells (E:T=100:1) which had been transiently transfected with either green fluorescent protein (GFP), (PEGFP; Clontech, Palo Alto, California) as a control or GFP plus pFAP plasmid encoding murine FAP. After about 48 hours, nuclear apoptosis was assessed as described above with the DNA-specific Hoechst 33342 dye (2 µM).

For the ⁵¹Cr release assay Balb/c mice (n=3) were treated four times at weekly intervals. Splenocytes were harvested 13 days after the last treatment and incubated for 5 days with γ-irradiated (about 1000 Gy, 45 minutes) A31 fibroblasts (ATCC, Manassas, Virginia), which were retrovirally infected with pFap. Stimulated splenocytes were then incubated for about 4 hours with labeled A31-pFap and the percentage of lysis was calculated. Cells were also co-incubated with anti-MHC class I antibodies (BD Biosciences, Rockville, Maryland) at a concentration of about 10 µg/ml.

**Immunohistochemistry.** Cryosections (about 8 µm thickness) were fixed in acetone and strained. After incubation with the primary antibody (either a polyclonal rabbit anti-murine FAP antibody (provided by Dr. J. D. Cheng), or a polyclonal rabbit anti-murine collagen type I antibody (Chemicon, Temecula, California), the sections were immunostained according to the manufacturer's protocol (DAKO LSAB+ Kit, Peroxidase, DAKO, Carpinteria, California).

For focal microscopy, fixed cryosections were stained with anti-murine CD8 antibody, biotinated anti-rat Ig secondary antibody, FITC labeled Streptavidin (BD Bioscience, Rockville, Maryland) and co-stained with DAPI (Sigma, St. Louis, Missouri). A laser scanning confocal microscope (LSCM) was used to obtain images, which were processed using Zeiss Image Examiner software (Carl Zeiss). For FACS analysis of T-cell infiltration Balb/c mice (*n*=6) were treated three times at weekly intervals with either pFap or empty vector. One week after the last treatment animals were challenged s.c. with about 3x10⁵ CT26 tumor cells. Tumors were harvested 3 weeks later and single cell suspensions prepared by incubating sliced tumor tissue for 45 minutes in medium supplemented with collagenase type I (125U/ml; GIBCO, Gaithersburg, Maryland). After filtering, cells of two mice were pooled, stained with anti-CD3⁺ PerCp-Cy5.5 and anti-CD8⁺ FITC (BD Biosciences, Rockville, Maryland) and analyzed by FACS.

**Intratumor uptake of fluorescein, Evans blue albumin, and ¹⁴C-5-fluorouracil.** After the last of three treatments at 1-week intervals with either control vaccine (Example 1), DNA Composition 1 (Example 1) or DNA Composition 2 (Example 2), mice were challenged 10 days later by subcutaneous injection of about 3x10⁴ CT26 cells in the left front flank. About 19 days thereafter, mice were given intrapertioneal (i.p.) injections of either 1% fluorescein sodium (Sigma) at about 12 µl/g of body weight, i.v. injection of about 100 µl Evans blue albumin (Sigma), or i.v. injection of about 2.5 µCi of ¹⁴C-5-fluorouracil (Sigma). After about 5 minutes, 30 minutes or 1 hour, respectively, mice were sacrificed to determine absorption or scintillation of the supernatants of tumor homogenates at 490 nm, 612 nm, or in a γ-counter, respectively.

For the determination of intratumoral doxorubicin uptake Balb/c mice (*n*=4) were challenged s.c. with about 5x10⁵ D2F2 cells in the right flank. Doxorubicin was injected i.v. 16 days later (10 mg/kg) and tumors were harvested 45 minutes thereafter. Samples were measured against the internal standard daunorubicin using an Eclipse XCB-C8 column on a 1100 series LC-MS (Agilent, Foster City, California).

**Evaluation of potential side effects.** In order to determine any detrimental effects on wound healing, mice were surgically wounded. A circular wound of about 3 mm in diameter was made with a dermal punch (Miltex Inc., Bethpage, New York) on the upper back of BALB/c mice (*n*=4) about 10 days after the last of 3 treatments at 1-week intervals. The time required for wound closure was measured. Despite the fact that FAP is overexpressed during wound healing, the compositions of the present invention do not interfere with the wound healing process. After inflicting a circular wound of about 3 mm diameter on the backs of treated BALB/c mice (*n*=4), no significant differences in wound healing was observed between treated and non-treated mice. For histological analysis wounds were inflicted on treated mice 7, 14, 21 days before examination. Skin biopsies, as well as 26 organs and tissues were examined by a mouse pathologist.

**Prophylactic treatment with the FAP-based DNA-composition inhibits primary tumor growth.** Ten days after the last of three treatments at one week intervals with either control vaccine (Example 1), DNA Composition 1 (Example 1) or DNA Composition 2 (Example 2), different groups of BALB/c mice (*n*=8) were challenged either subcutaneously with CT26 colon carcinoma cells or orthotopically with D2F2 breast carcinoma cells as described above. The DNA compositions of the invention suppressed primary tumor growth of multi-drug resistant CT26 cells (FIG. 2*a*), as well as the resistant D2F2 cells (FIG. 2b). DNA Composition 2, which encodes for a combination of the FAP, CCL21, and IL-2, were about equally effective in inhibiting primary tumor growth (Fig 2*a*).

**A DNA composition of the invention reduces growth of established metastasis in a therapeutic setting.** BALB/c mice treated with DNA Composition 1 of Example 1 three and ten days after an initial intravenous inoculation with about 1x10⁵ CT26 colon carcinoma cells resulted in a significant reduction in experimental pulmonary metastases compared to mice treated with control vaccine of Example 1. In contrast, mice in the control group exhibited extensive metastases and began to die 18 days after tumor cell inoculation (FIG. 2c). Similarly, a combination of pFAP with pCCL21 alone was also about equally effective (FIG. 2b).

**CD8⁺ T cells provide an effective anti-tumor immune response.** Mice were treated i.v. three times at 1-week intervals with DNA Composition 1 (Example 1) and challenged with about 1x10⁵ CT26 cells. The mice were then depleted of their respective effector cells during the effector phase with antibodies against CD4⁺ or CD8⁺ T cells, as well as NK cells (FIG. 3, Panel A). Depletion of CD4⁺ cells and NK cells did not decrease the effectiveness of the pFAP treatment, indicating an important role for CD8⁺ T cells in the immune response.

To assess the extent to which treatment with the FAP DNA compositions of the invention is able to break peripheral T cell tolerance against the FAP self-antigen, CD8⁺ T cells from animals treated with bacteria transfected with pFAP or the empty vector were purified. These cells were then stimulated with γ-irradiated tumor target cells, and incubated with live tumor target cells that were transiently transfected with either GFP as a control or GFP/pFap. As shown in FIG. 3, Panel B, only CD8⁺ T cells purified from mice treated with pFap were able to induce nuclear apoptosis as assessed by staining of pFap transfected target cells with Hoechst-33342 dye.

Splenocytes of treated mice were also used in a conventional ⁵¹Cr-release assay. To this end, splenocytes from mice treated with Composition 1 and the control vaccine were incubated for 5 days with γ-irradiated A31 fibroblasts, which were retrovirally infected with pFap. Stimulated splenocytes were then incubated for 4 hours with labeled A31-pFap cells and the percentage of lysis was calculated. Splenocytes from mice treated with Composition 1 were able to lyse significantly more fibroblasts than those from empty vector controls at target-to-effector ratios of 1:100 and 1:25 (FIG. 3, Panel C). Co-incubation with anti-MHC class I antibodies eliminated this effect (FIG. 3, Panel D).

In an additional evaluation, mice were treated three times with either Composition 1 or the control vaccine and then challenged with about 3x10⁴ CT26 tumor cells to investigate CD8⁺ T-cell infiltration in tumors of pFap treated mice. After three weeks, tumors were harvested and single cell suspensions stained for CD3⁺ and CD8⁺ cells and analyzed by FACS. Mice treated with Composition 1 showed a marked increase in CD3⁺CD8⁺ cells in the tumor tissue when compared to the empty vector control (FIG. 3, Panel E).

Tumor sections were also stained with anti-CD8 FITC and DAPI nuclear stain. Using confocal microscopy, tumors of mice treated with Composition 1 showed a more marked infiltration with CD8⁺ cells than mice treated with the control vaccine of Example 1 (FIG. 3. Panel F). Taken together, these findings demonstrate that a DNA composition of the invention, targeting FAP, can overcome peripheral T cell tolerance against the FAP self-antigen.

**Suppression of collagen type I expression increases intratumor dye uptake.** Fibroblasts are the primary source of collagen type I and it has been reported that expression of this molecule correlates inversely with intratumoral uptake of compounds of varying molecular weights. To evaluate whether this same mechanism is applicable to the DNA compositions of the invention, tumor sections of treated mice were stained with antibodies against FAP (FIG. 4, Panel A, upper photos) or against collagen type I (FIG. 4, Panel A, lower photos). A decrease in the expression of FAP as well as collagen type I was detected in groups of mice treated with Composition 1 as compared to mice treated only with the control vaccine (Example 1). Corresponding western blots of these tumor extracts, stained with the same antibodies, revealed about 82.63 +/- 2.54% decrease in FAP expression (FIG. 4, Panel B, upper photo) and about 76.36 +/- 2.01% decrease in collagen type I expression (FIG. 4, Panel B, lower photo).

Mice were then injected with three compounds differing in size and structure , i.e. fluorescein (376 Da) (FIG. 4, Panel C), Evans blue albumin (68,500 Da) (FIG. 4, Panel D) or the chemotherapeutic agent 5-fluorouracil (130 Da) (FIG. 4, Panel E) as described above. Tumors of mice treated with pFap DNA Composition 1 incorporated significantly more (p<0.05) of these respective molecules than those of mice administered with the control vaccine.

**Combination of DNA composition and chemotherapy leads to tumor-rejection.** A therapeutic protocol was developed combining treatment with a DNA composition of the invention with the chemotherapeutic drug doxorubicin, to which the D2F2 cells were partially sensitive (FIG. 1, Panel C). BALB/c mice (*n*=8) were treated with the control vaccine of Example 1, or Composition 1 of Example 1. The treated mice were then challenged orthotopically with D2F2 breast carcinoma cells. These two groups of mice were then treated with either doxorubicin or PBS as a control 5, 10 and 15 days after tumor challenge. As shown in FIG. 5, Panel A, single treatments with either immunotherapy (Composition 1) or chemotherapy were only able to suppress, but not eradicate tumor growth. In contrast, the group of mice treated with Composition 1 and doxorubicin in combination not only revealed a marked inhibition of tumor growth, but also a complete tumor-rejection in 4 of 8 mice (FIG. 5, Panel A).

In another combination therapy approach in a therapeutic setting, BALB/c mice (*n*=8) were challenged i.v. with D2F2 tumor cells. Once metastasis had been established after 5 days, the mice were treated weekly with Composition 1. One day after each treatment the mice were injected i.v. with doxorubicin. As a result of this combination treatment the life span of these mice exceeded more than 3 times that of control mice that were treated with doxorubicin or Composition 1 alone, and died already after about 35-45 days (FIG. 5, Panel B).

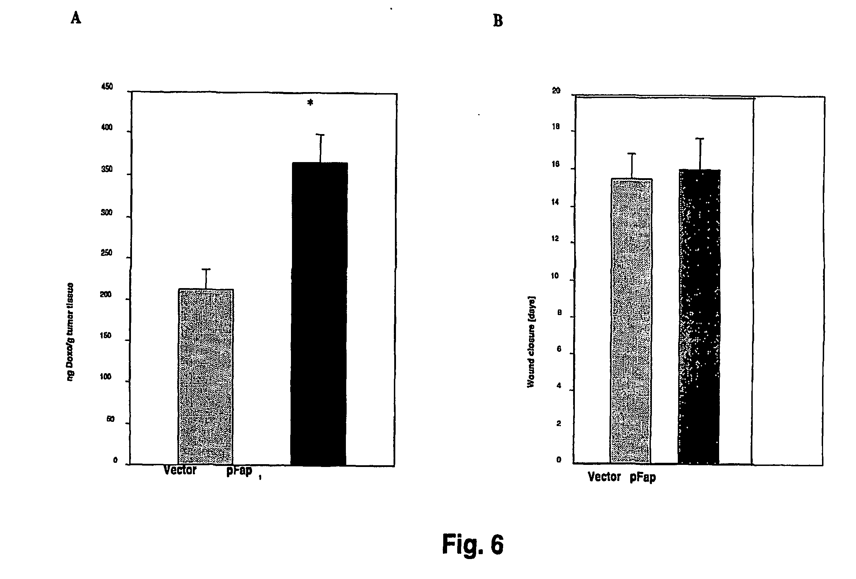

**A FAP DNA composition of the invention increases intratumoral uptake of doxorubicin.** To determine the doxorubicin concentration in the tumor tissue, mice (*n*=4) were treated three times at 1-week intervals with Composition 1, challenged 7 days later with 5x10⁵ D2F2 tumor cells, and injected 16 days later i.v. with doxorubicin. The intratumoral drug concentration was determined with liquid chromatography /mass spectrometry (LC-MS). Tissues of mice treated with Composition 1 showed a significant increase of doxorubicin uptake in the tumor (FIG. 6, Panel A) compared to controls. These results are in line with the observed chemical infiltration of tumors in treated mice with fluorescein, albumin, and 5-fluorouracil (FIG. 4, Panels C-D).

**The DNA vaccine against FAP does not impair wound healing or damage normal tissue.** Since FAP is overexpressed during wound healing, the effects of the DNA compositions of the invention on wound healing were investigated. A circular wound of about 3 mm in diameter was made on the backs of treated BALB/c mice (n=4). Surprisingly, no significant difference in wound healing between treated and non-treated mice was observed (FIG. 6, Panel B). Histological assessment by a mouse pathologist of these wounds after different time points revealed no qualitative abnormalities in the wound healing process. To exclude any autoimmune reactions induced by the DNA compositions of the invention, comprehensive histology of the following tissues and organs was performed: skin, brain, spinal cord, muscle, bone, synovium, heart, aorta, pulmonary artery, thymus, spleen, lymph nodes, bone marrow, parathyroid, adrenal gland, kidney, uterus, vagina, clitoral gland, tongue, liver, lungs, pancreas, stomach, small intestine and colon. No discernable differences were observed compared to control mice.

## Claims

1. A composition effective for eliciting an immune response against tumor stromal cells that express fibroblast activation protein (FAP) comprising a DNA construct that encodes for FAP and is incorporated in an attenuated *Salmonella typhimurium* bacterial vector, wherein the DNA construct is expressible in immune cells, and is incorporated in a pharmaceutically acceptable carrier.

2. The composition of claim 1 wherein the DNA construct encodes for human FAP (SEQ ID NO: 2).

3. The composition of claim 1 wherein the DNA construct is a substantially purified DNA having the polynucleotide sequence consisting of SEQ ID NO: 1 or a polynucleotide sequence that has at least about 80% sequence similarity therewith.

4. The composition of claim 1 wherein the composition further comprises a DNA construct encoding an immune effector protein expressible in immune cells.

5. The composition of claim 4 wherein the immune effector protein is a cytokine.

6. The composition of claim 5 wherein the cytokine is CCL21, IL-2, or CD40LT.

7. The composition of any one of claims 1-6 for use in inhibiting tumor growth or tumor metastases.

## Patentansprüche

1. Zusammensetzung, die für ein Auslösen einer Immunreaktion gegen Tumor-Bindegewebszellen, die Fibroblasten-Aktivierungsprotein (FAP) exprimieren, wirksam ist und ein DNA-Konstrukt umfasst, das für FAP kodiert und in einen attenuierten *Salmonella typhimurium-*Bakterienvektor eingebaut ist, wobei das DNA-Konstrukt in Immunzellen exprimierbar ist und in einen pharmazeutisch verträglichen Träger eingebaut ist.

2. Zusammensetzung nach Anspruch 1, wobei das DNA-Konstrukt für menschliches FAP (SEQ ID NO: 2) kodiert.

3. Zusammensetzung nach Anspruch 1, wobei das DNA-Konstrukt eine im Wesentlichen aufgereinigte DNA ist, die die Polynukleotidsequenz, die aus SEQ ID NO: 1 besteht, oder eine Polynukleotidsequenz, die mindestens etwa 80% Sequenzähnlichkeit damit hat, aufweist.

4. Zusammensetzung nach Anspruch 1, wobei die Zusammensetzung ferner ein DNA-Konstrukt umfasst, das für ein Immuneffektorprotein kodiert, das in Immunzellen exprimierbar ist.

5. Zusammensetzung nach Anspruch 4, wobei das Immuneffektorprotein ein Cytokin ist.

6. Zusammensetzung nach Anspruch 5, wobei das Cytokin CCL21, IL-2 oder CD40LT ist.

7. Zusammensetzung nach einem der Ansprüche 1 bis 6 zur Verwendung bei einer Hemmung von Tumorwachstum oder Tumormetastasen.

## Revendications

1. Composition efficace pour déclencher une réponse immune contre les cellules de stroma tumorales qui expriment une protéine d'activation des fibroblastes (FAP), comprenant une construction d'ADN qui code pour la FAP et est incorporée dans un vecteur bactérien de *Salmonella typhimurium* atténué, tandis que la construction d'ADN est apte à être exprimée dans des cellules immunes, et est incorporée dans un support pharmaceutiquement acceptable.

2. Composition selon la revendication 1, dans laquelle la construction d'ADN code pour la FAP humaine (SEQ ID NO: 2).

3. Composition selon la revendication 1, dans laquelle la construction d'ADN est un ADN substantiellement purifié ayant la séquence polynucléotidique consistant en SEQ ID NO: 1 ou une séquence polynuélcotidique qui a au moins environ 80% de similarité de séquence avec elle.

4. Composition selon la revendication 1, dans laquelle la composition comprend en outre une construction d'ADN codant pour une protéine effectrice immune apte à être exprimée dans des cellules immunes.

5. Composition selon la revendication 4, dans laquelle la protéine effectrice immune est une cytokine.

6. Composition selon la revendication 5, dans laquelle la cytokine est CCL21, IL-2, ou CD40LT.

7. Composition selon l'une quelconque des revendications 1-6 pour utilisation dans l'inhibition de la croissance tumorale ou des métastases tumorales.
